# EUROPEAN PATENT SPECIFICATION

(11) **EP 0 812 421 B1**
(45) Date of publication and mention of the grant of the patent: **19.06.2002**
(21) Application number: 96903036.0
(22) Date of filing: 29.02.1996
(51) Int. Cl.: G01N 33/564, C07K 1/22, C07K 16/18, A61K 31/70, A61K 39/395

(54) **ANTI DNA ANTIBODY DETECTION INVOLVING TELOMERIC DNA SEQUENCE RECOGNITION AND BINDING**
ANTI-DNS-ANTIKÖRPERNACHWEIS MITTELS ERKENNUNG UND BINDUNG TELOMERISCHER DNS-SEQUENZEN
DETECTION D'ANTICORPS ANTI-ADN IMPLIQUANT LA RECONNAISSANCE ET LA FIXATION DE SEQUENCES D'ADN TELOMERES

(30) Priority: 01.03.1995 FI 950962
(43) Date of publication of application: 17.12.1997
(73) Proprietor: BIOHIT OYJ, 00880 Helsinki (FI)
(72) Inventor: Salonen, Eava-Marjatta, 00100 Helsinki (FI)
(74) Representative: Grew, Eva Regina
(86) International application number: FI9600117
(87) International publication number: WO9627131

(56) References cited:
- EP-A- 0 205 643
- EP-A- 0 276 984
- WO-A-93/23572
- PROC. NATL. ACAD. SCI. U.S.A., Volume 92, March 1995, S.M. BARBAS et al., "Human Autoantibody Recognition of DNA", pages 2529-2533.
- PROC. NATL. ACAD. SCI. U.S.A., Volume 92, January 1995, HERREN WU et al., "Building Zinc Fingers by Selection: Toward a Therapeutic Application", pages 344-348.
- NATIONAL OF LIBRARY OF MEDICINE, FILE MEDLINE, NLM Accession No. 95031010, GABIBOV A.G. et al., "DNA-Hydrolyzing Autoantibodies"; & APPL. BIOCHEM. BIOTECHNOL., May-Jun. 1994, 47(2-3), 293-302, Discussion 303.
- J. AM. CHEM. SOC., Volume 116, 1994, S.M. BARBAS et al., "Recognition of DNA by Synthetic Antibodies", pages 2161-2162.
- DIALOG INFORMATION SERVICES, DERWENT DATABASE WPI, Dialog Accession No. 009259776, WPI Accession No. 92-387189/47, TOSHIBA K.K., "Method of Detecting Gene by Reacting Sample With Antibody to Single Stranded Oligo-Nucleotide - of Given Base Sequence, Then Detecting Presence of Antigen-Antibody Reaction"; & JP,A,04 286 957, (12-10-92), 9247 (Basic).
- NATIONAL LIBRARY OF MEDICINE, FILE MEDLINE, NLM Accession No. 95058242, SCHRIEVER-SCHWEMMER G. et al., "Differentiation of Micronuclei in Mouse Bone Morrow Cells: a Comparison Between CREST Staining and Fluorescent in Situ Hybridization With Centromeric and Telomeric DNA Probes"; & MUTAGENESIS, Jul. 1994, 9(4), 333-40.
- NATIONAL LIBRARY OF MEDICINE, FILE MEDLINE, NLM Accession No. 95347387, HERRMANN M. et al., "Preferential Recognition of Specific DNA Motifs by Anti-Double-Stranded DNA Autoantibodies"; & EUR. J. IMMUNOL., Jul. 1995, 25(7), 1897-904.

### SEQUENCE LISTING

(1) GENERAL INFORMATION:
   (i) APPLICANT:
      (A) NAME: Salonen, Eeva-Marjatta
      (B) STREET: Tunturikatu 15 B
      (C) CITY: Helsinki
      (E) COUNTRY: Finland
      (F) POST CODE (ZIP): 00100
   (ii) TITLE OF INVENTION: Diagnostic Method, Test Kit, Drug and Therapeutic Treatment for Autoimmune Diseases
   (iii) NUMBER OF SEQUENCES: 2
   (iv) CORRESPONDENCE ADDRESS:
      (A) ADDRESSEE: Borenius & Co Oy Ab
      (B) STREET: Arkadiankatu 21 A
      (C) CITY: Helsinki
      (E) COUNTRY: Finland
      (F) POST CODE (ZIP): 00100
   (ix) TELECOMMUNICATION INFORMATION;
      (A) TELEPHONE: +358-0-492593
      (B) TELEFAX: +358-0-493542
      (C) TELEX: 124260 boco fi
(2) INFORMATION FOR SEQ ID NO: 1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: CDNA
   (xi) SEQUENCE DESCRIPTION: SEQ ID NO: 1:
   TTAGGGTTAG GGTTAGGGTT AGGGTTAGGG 30
(2) INFORMATION FOR SEQ ID NO: 2:
   (i) SEQUENCE CHARACTERISTICS:
      (A) LENGHTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 2:

## Description

### FIELD OF INVENTION

The present invention relates to a method for detecting anti-DNA-antibodies in samples obtained from eukaryotic organisms, especially in vertebrate body fluids, and more particularly to a diagnostic method for detecting anti-DNA-antibodies in body fluids from human beings and domestic animals. The invention also relates to test kits for performing the said method and to drugs and therapy for patients suffering from diseases involving the presence of anti-DNA-antibodies, specifically autoimmune disorders such as Lupus Erythematosus and Rheumatoid Arthritis.

### BACKGROUND OF THE INVENTION

Lupus erythematosus is an autoimmune disorder (antibodies are produced against self antigens), in which the body's immune system, for unknown reasons, attacks the connective tissue as though it were foreign, causing inflammation. Of the multitude of autoreactive antibodies that spontaneously arise during the disease, high levels of circulating autoantibodies to DNA are the best evidence of the pathogenesis.

In Systemic Lupus Erythematosus (SLE) there is almost invariable presence in the blood of antibodies directed against one or more components of cell nuclei. Certain manifestations in SLE seem to be associated with the presence of different antinuclear antibodies and genetic markers, which have suggested that SLE may be a family of diseases [Mills, J.A., Medical Progress 33, 1871-1879 (1994)].

The more common type of lupus erythematosus, Discoid Lupus Erythematosus (DLE), affects exposed areas of the skin. The more serious and fatal form, Systemic Lupus Erythematosus (SLE), affects many systems of the body, including the joints and the kidneys.

Animal models have confirmed that organ damage and premature death occurs only following the skewing of the B cell repertoire towards autoreactivity [Klinman, D.M. J.Clin. Invest. 86, 1249-1254 (1990). Klinman, D.M. et al., J.Immunol. 144, 506-511 (1990)]. Lupus nephritis, especially diffuse proliferative glomerulonephritis, has been known to be associated with circulating antibodies to double-stranded (native) DNA [Casals, S. et al., Arthritis Rheum. 7, 379-390 (1964); Tan E.M. et al., J.Clin. Invest. 82, 1288-1294 (1966)]. The detection of antinuclear antibodies is a sensitive screening test for SLE. Antinuclear antibodies occur in more that 95 % of patients IHochberg, M.C. Rheum. Dis. Clin. North Am. 16, 617-39 (1990)].

The most common antibody in patients with SLE is directed against nucleosomal DNA-histone complexes, and it yields a homogeneous staining pattern on the immunofluorescence test for antinuclear antibodies [Mohan, C. et al., J. Exp. Med. (1993) 177, 1367-81]. Antinuclear antibodies are also seen in most of the other rheumatic diseases, [Tan, E.M., Adv. Immunol. (1989) 44, 93-151] and are produced transiently in viral infections and are present, usually in low titers, in about 2 percent of the normal population.

Antibodies to native or double-stranded DNA and to Sm, a ribonuclear protein antigen, are more specific than other antinuclear antibodies for the diagnosis of SLE. [Mills, J.A., Medical Progress 33, 1871-1879 (1994)]. It is more common to have nephritis in patients with antinative DNA, the titer of this antibody being a useful measure of disease activity [Swaak, A.J.G. et al., Ann. Rheum. Dis. 1986, 45: 359-66; ter Borg, E.J. et al., Arthritis Rheum. 1990: 33, 634-43].

The 10-year average survival rate from diagnosis for patients with SLE observed over the past decade approaches 90 percent [Pistiner et al., Semin. Arthritis Rheum. 1991: 21, 55-64; Ginzler et al., Rheum. Dis. Clin. North Am. 1988: 14, 67-78].

During 1993-1994 the present inventor was working at the Scripps Research Institute (La Jolla, CA, USA) in a project involving a human antibody gene library from a Systemic Lupus Erythematosus (SLE) donor. The library was panned against human placental DNA (a commercial product), and selected monoclonal antibodies were tested for human antibody recognition of DNA. The results of the project are disclosed in Barbas, S.M., Ditzel, H.J., Salonen, E.-M., Yang, W.-P., Silverman, G.J. and D.R. Burton, Human autoantibody recognition of DNA, Proc. Natl. Acad. Sci. 1995: 92, 2529-2533. The object was to discover a specific sequence of DNA that would be recognized by a human SLE autoantibody.

A haploid set of human chromosomes (haploid human genome) is composed of the estimated 3 billion (3×10⁹) bp of DNA. The probability of finding, experimentally, a short sequence that is recognized by autoantibodies is small. Thus, despite a vast amount of work, a functionally correct sequence has not been found.

### SUMMARY OF THE INVENTION

Subsequently, the present inventor in her studies in another context has unexpectedly found that synthesized telomeric sequences, i.e. sequences that are found at the terminal ends of the chromosomes bind the autoantibodies in question and that they can be used to detect and to inhibit said anti-DNA-antibodies.

It is therefore an object of the present invention to provide a method for detecting anti-DNA-antibodies in eukaryotic samples.

More specifically, it is an object of the invention to provide a method for detecting anti-DNA-antibodies in body fluids such as serum or cerebrospinal fluid from vertebrates, especially human beings.

It is a further object of the invention to provide a diagnostic method for the detection of Lupus Erythematosus, Rheumatoid Arthritis, Scleroderma and other autoimmune diseases, especially Systemic Lupus Erythematosus in vertebrates, by detecting an elevated level of anti-DNA-antibodies in a body fluid sample of said vertebrate with the aid of telomeric sequences specific to said mammal and/or with the aid of antibodies or fragments of antibodies specific to said telomeric sequence.

In the following specification and claims the term "telomeric sequence" used generally in context with the invention should be understood as meaning any of the telomeric sequences of DNA selected from the group consisting of a single-stranded vertebrate telomeric sequence, a complementary strand thereto, a double stranded vertebrate telomere, and a part or a repeat or a combination of any of the foregoing, which is capable of binding said anti-DNA-antibodies.

The term antibody specific to said telomeric sequence should be understood as meaning any antibody which specifically binds to said telomeric sequence and hence Is capable of competition with said anti-DNA-antibody. Said specific antibody may be of any lg class, but it is preferably an antibody of the IgG, IgM or IgA class. Special benefits of the invention are obtained by the use of fragments of the specific antibody, especially Fabs or F(ab')₂s. Said fragments may be derived from naturally occurring anti-DNA-antibodies or they may preferably be produced by recombinant DNA techniques.

It Is also an object of the invention to provide a test kit for detecting anti-DNA-antibodies, said test kit preferably including immobilized telomeric sequences capable of binding to anti-DNA-antibodies. The test kit preferably includes a label indicating the binding of anti-DNA-antibody to telomeric sequence.

An object of the invention is to provide both quantitative and qualitative test kits for the detection of anti-DNA-antibodies.

It is also an object of the invention to provide a means for the removal of anti-DNA-antibodies from a liquid sample, especially from a body fluid of a vertebrate, such as the serum of a human being by selectively binding said anti-DNA-antibodies to telomeric sequences outside the body of said vertebrate. Said means preferably comprises a column or matrix including immobilized telomeric sequences capable of binding anti-DNA-antibodies.

It is a further object of the invention to provide a drug capable of inhibiting the binding or reducing the activity of anti-DNA-antibodies in patients suffering from autoimmune disorders, which drug contains an amount of telomeric sequences or fragments of antibodies thereto effective in inhibiting the telomeric sequence binding or neutralizing the destructive action of the patient's specific anti-DNA-antibodies.

Telomeres, terminal DNA-pratein complexes of chromosomes, are essential in the protection, positioning and for the stability of chromosomes [E.H. Blackburn and J.W. Szostak, Annu. Rev. Biochem. 53, 163 (1984); V.A. Zakian, Annu. Rev. Genet. 23, 579 (1989)]. They also are required for the complete replication of the chromosomal terminus during each cell cycle. All known eukgryotic telomeres consist of hundreds to thousands of simple, repeated tandem sequences of DNA and associated proteins. A functional human telomere is defined as having a repeat of the sequence 5'-TTAGGG-3' [E.H. Blackburn, Nature 350, 568 (1991); R.K. Moyzis et at., Proc. Natl. Acad. Sci. U.S.A. 85, 6622 (1988); J. Meyne et al. Proc. Natl. Acad. Sci. U.S.A. 86, 7049 (1989)]. All vertebrates have the same telomeric sequence (TTAGGG)ₙ [Moyzis, R.K., Buckingham, J. M., Cram, L.S., Dani, M., Deaven, L.L., Jones, M. D., Meyne, J., Ratliff, R. L. & Wu, J.-R. (1988) *Proc. Natl. Acad. Sci. USA* 85, 6622-6626, J. Meyne, R. L. Ratliff, R. K. Moyzis, *Proc. Natl. Acad. Sci U.S.A* 86, 7049 (1989)]. A general structure of repeated sequences of G- and C-rich complementary strands of the eukaryotic telomeres is (T or A)ₘ(G)ₙ [Blackburn, E.H. and Szostak, J.W. Annu, Rev. Biochem. 53, 163-194 (1984); Weiner, A.M. Cell 52, 155-157 (1988)].

Telomeres can be visualized at the tips of the chromosomes using a fluorescence microscope. When labeled (GGGTTA)₇'(TAACCC)₇ oligomers were hybridized to metaphase chromosomes using a biotin-avidin detection method the distinct, speckled fluorescent signal was distinctly seen at the chromosomal ends [Meyne, J.M. et al., Proc. Natl. Acad. Sol. 86, 7049-7053 (1989)]. Although telomeric sequences are highly conserved in evolution, the correct sequence of the telomeric repeat is required for telomere function, since, for example, addition of telomeric DNA harboring a mutated telomeric sequence to the ends of the endogenous Tetrahymena telomeres (having a sequence repeat 5'-TTGGGG-3') leads to telomere length instability and death [Yu et al., Nature (London) 344, 126-132 (1990); Harley, C.B. et al., Nature (London) 345, 458-460 (1990)].

The structure and function of telomeres has been described by E.H. Blackburn, Nature, 350, 569-573 (1991). Telomeres have not been reported as having any connection with SLE or other autoimmune diseases.

For better understanding of the present invention, together with other and further objects and the nature and advantages of the invention, reference is made to the following detailed description of specific embodiments.

### DETAILED DESCRIPTION OF THE INVENTION

The present invention is based on the novel finding that autoantibodies (anti-DNA-antibodies) are recognized and bound by telomeres. In accordance with the present invention, a method is disclosed for the detection of autoantibodies or anti-DNA-antibodies in samples of eukaryotic origin, especially vertebrate body fluids, especially serum.

Body fluids which may contain anti-DNA-antibodies in addition to serum are, for instance, cerebrospinal fluid, synovial fluid, pleural fluid, ascites, saliva, tears, urine, intestinal secretions, lymph fluid, etc. The IgG, IgM and IgA class antibodies are known to be secreted into the bloodstream, while mainly IgA antibodies are found in saliva, tears, etc. The preferred body fluid for quantitative detection according to the invention is mammalian serum.

Telomeres are evolutionally highly conserved in nature and all eukaryotic beings have telomeres of a very closely related structure. The vertebrate telomere is composed of repeated sequences of G- and C-rich complementary strands with a G-rich strand that extends past the rich strand as 3' overhang. Other eukaryotic telomeres have very similar sequences, usually only one nucleotide in the hexapeptide is different. Thus, the present invention is applicable on humans and animals alike.

It is also possible to use parts of the telomere that recognize the anti-DNA-antibodies as binding partners. Thus, a part of the hexanucleotide repeat, such as the TTAG, may be used for performing the recognition.

It has been found that the anti-DNA-antibodies in some samples may bind more specifically to one or the other of the complementary single-stranded sequences, while some bind more readily to a double-stranded telomeric DNA (dsDNA).

It is also possible to use specific antibodies to said telomeric sequence for competing with the binding reaction between telomere and sample anti-DNA-antibody. Specifically light chain fragments, Fabs of said specific antibodies may be used for competing with said anti-DNA-antibodies.

The telomeric sequence used in the present invention is either the single-stranded telomere sequence, which for vertebrates is 5'-TTAGGG-3', its complementary sequence (for vertebrates 5'-CCCTAA-3'), a functional part of any of the above, an oligonucleotide having one or more repeats of the telomeric or complementary sequence, a double-stranded DNA formed of the above single-stranded sequences or an oligonucleotide part thereof or repeats of the telomeric doublet.

In the method for detecting anti-DNA-antibodies according to the present invention, the selected functional telomeric sequence is synthetized in a way known to those skilled in the art, e.g. in commercial nucleotide synthetizers. The obtained sequence is then preferably immobilized on a carrier, which may comprise the walls of microtiter wells, glass or plastic beads, porous sheets, Sepharose etc. which carriers are well known to those skilled in the art.

Immobilization (solid-phase) techniques are also well known to those skilled in the art. The telomeric sequence may, for instance, be coated onto the walls of microtiter wells, glass or plastic beads, impregnated into porous sheets, filter papers or the like. The immobilization may be performed directly on the material in question or, preferably, by the use of a streptavidin-biotin system, which provides a stronger and more accurately oriented immobilization of the telomeric sequence. The telomeric sequences may be biotinylated either at the 3' or at the 5' end. However, the actual technique used for the immobilization is not critical to the performance of the invention. The amount of telomeric sequence used is between 7-2 µg/ml, or even considerably more. The amount of telomeric sequence is not critical to the performance of the assay. A preferred range is believed to be 100-500 ng/ml.

The specific telomeric antibody and the fragments thereof may be provided from the serum of patients suffering from autoimmune diseases with anti-DNA-antibodies in ways known to those skilled in the art, or they may be provided by recombinant DNA techniques. Thus, the telomeric sequences in accordance with the present invention can be used for the production of anti-DNA antibodies and fragments thereof for therapeutic use.

When an immobilized telomeric sequence is contacted with a sample to be tested, any anti-DNA-antibodies present in the sample will attach to the immobilized sequence. The complex formed in the binding reaction may be detected by means well known in immunochemistry. Correspondingly, specific telomere antibodies or Fab fragments thereof may be used in a competitive immunochemical assay to indicate the presence of anti-DNA-antibodies in a sample.

The autoantibodies recognized by the telomeric sequences may be of different immunoglobulin classes, which may indicate so far unknown differences in the progression of the disease or disease pattern. Thus, the autoantibodies may be either IgG, IgM, IgA, IgD, or IgE class, since the anti-DNA-antibodies can belong to any of the known immunoglobulin classes.

Different anti-Ig antibodies will detect the presence of the different Ig class autoantibodies. Thus, for example, labeled anti-IgG will be used to determine the presence of the IgG class autoantibodies, anti-IgM antibody will be used for detecting the IgM class autoantibodies, and so on.

It is known in the art that SLE patients have predominantly IgG class autoantibodies, while for instance rheuma patients seem to have a predominance of IgM autoantibodies in their serum. However, the tests performed in connection with the present invention show that SLE patients often have either IgG, IgM or IgA class autoantibodies, the level of IgG class autoantibodies being elevated in 50 of 52 tested patients known to have SLE, compared to SLE negative patients. The two patients having rather low levels of IgG class antibodies were over 70 years of age and when IgG, IgM and IgA class antibodies were tested, all 52 SLE patients gave positive test results with the diagnostic test of the invention.

The bound anti-DNA-antibody can be detected with the aid of a label on an anti-Ig antibody used in a way known in the art, the sensitivity of the test depending on the label, the immunoassay, etc. The label may be a radioactive label, an enzyme label, a fluorochrome or fluorescence label, a dye, a sol, biotin, a luminescent label, a colored latex particle and/or a labeled polyclonal or monoclonal antibody or the like. The telomeric sequence or a Fab or F(ab')₂ fragment may also be labeled and used in known types of competitive immunoassays. The assay may be an EIA, radioimmunoassay, immunofluorescent assay, immunoprecipitation, complement fixation, immunochromatographic assay or any other that is designed to detect autoantibody binding of a telomeric DNA.

The test may be formulated as a quantitative test such as an EIA assay or into a more or less qualitative rapid test, as desired, using procedures which are known *per se* in the art and which are exemplified in the Examples hereinafter.

The tests performed in connection with the present invention very conclusively show that a diagnosis of systemic lupus erythematosus patients can be performed using a telomeric sequence for the anti-DNA-antibody recognition. Out of 52 SLE patients all were diagnosed positive in at least one Ig class autoantibody binding when tested with the assays of the present invention. The tests of the present invention are easy to perform, they are quick and reliable. The need for this kind of test is enormous in the art, since previously the testing for SLE has been uncertain and tedious. In actual fact no certain diagnosis has been possible until clear signs of serious disorders have appeared.

The tests performed also show that patients with rheumatoid arthritis may be diagnosed by detecting the presence of anti-DNA-antibodies, especially in the IgM class.

Further, several psoriasis patients showed elevated levels of anti-DNA-antibodies compared to healthy controls.

In systemic lupus erythematosus (SLE) the serum of the patient almost invariably contains antibodies directed against the cell nuclei (anti-DNA-antibodies). Some of these are directly responsible for the disorders caused by the disease. According to the invention it has been found that telomeric sequences bind these autoantibodies. One aspect of the present invention therefore comprises the use of this binding affinity for removing anti-DNA-antibodies from body fluids, especially from the serum of humans.

The removal of anti-DNA-antibodies may be performed in an affinity column containing immobilized telomeric sequences capable of retaining anti-DNA-antibodies from a fluid sample. The fluid may at need be recirculated through the column. After use the bound anti-DNA-antibodies may be removed from the column by means known *per se* and the column may be reused. The treated fluid, such as human serum may be returned in a continuous fashion to the patient or it may be used for other purposes.

Removal of anti-DNA-antibodies from a fluid may also be achieved by using various batch processes with the telomeric sequences immobilized on a matrix of a suitable kind, such as plastic beads, filters, etc.

The frequency and the length of time needed for treatment of, for instance, the serum of a human being suffering from SLE by an affinity column or other anti-DNA-antibody removal means, may be monitored with a test designed according to the present invention.

It has been found that the telomeric sequences bind to the anti-DNA-antibodies and neutralize their binding capacity and that, correspondingly, antibody fragments specific to said telomeric sequences compete with the binding of anti-DNA-antibodies to said telomeric sequences and hence prevent the anti-DNA-antibodies from being bound to telomeres.

The telomeric sequences, parts or repeats thereof, single or double-stranded, and antibody fragments used in the above described assays may thus also be used as active agents in drugs against the destructive activity of the anti-DNA-antibodies in patients suffering from diseases and/or disorders caused by or involving the presence of anti-DNA-antibodies.

More specifically, the active part of the drug is a single or double-stranded telomeric sequence, a repeat or a part thereof, a complementary sequence or a combination of any of the foregoing, (referred to generally as a telomeric sequence) which is recognized by a patient anti-DNA-antibody, or a fragment (Fab or F(ab')₂) of an antibody specific to any of said telomeric sequences. A drug according to the present invention thus comprises an amount of a telomeric sequence or antibody fragment, as defined above, which is effective to inhibit or reduce the destructive activity of the patient's anti-DNA-antibodies.

Special benefits are provided by using the above antibody fragments in drugs since they will compete with autologous anti-DNA-antibodies for binding to the patient's telomeric sequences. The Fabs and F(ab')₂ fragments lack effector functions and will not activate the complement cascade leading to cell destruction when bound to an antigen, as the whole anti-DNA-antibodies do.

The present invention comprises drugs for humans as well as veterinary preparations containing the vertebrate telomeric sequence.

Moreover, the present invention makes it possible to give the patient in question precisely the telomeric sequence or specific antibody fragment that will be most effective to prevent the binding of the patient's own anti-DNA-antibodies to telomeric sequences. The patient can first be tested with an assay according to the present invention so as to determine which telomeric sequence will best bind to the anti-DNA-antibodies. Thus, assays will determine whether single or double-stranded telomeric sequences should be used, and which Ig class the patient's anti-DNA-antibodies are.

In this therapeutic embodiment of the present invention the regimen for therapy is preferably designed individually to each patient based on their anti-DNA-antibody binding, i.e which immunoglobulin class, IgG, IgM, IgA etc., and which telomeric form they prefer.

The amount of active telomeric agent that should be given to a patient will vary with the patient's race, age, illness, etc. and the most effective dose will have to be determined on a case by case basis. However, the telomeres are DNA sequences existing as such in the chromosomes and thus they are part of the mammalian body. Thus, it is not believed that the maximum amount of telomere given to a patient will be critical. In toxicity tests on rabbits no effect was observed after a telomere administration. The Fabs used according to the present invention will be of mammalian origin or they will be engineered by recombinant techniques to resemble the same and will therefore be acceptable to the mammalian body.

The active telomeric agent of the present invention may be formulated into pharmaceutical preparations such as injectable solutions in a way known in the pharmaceutical art.

The following Examples illustrate the invention without, however, limiting it in any way.

### Example 1

### Synthesis of telomeric sequences

Synthesis of oligonucleotides was carried out by a commercial apparatus, Oligonucleotide Synthesizer (Applied Biosystems). The following oligonucleotides were synthetized:

A duplex DNA (2667 + 2668) was formed in the laboratory as follows:

A volume of 50 µl (50µg) of each oligo (2667 and 2668) in 10 mM Tris- 1 mM EDTA containing 50 mM NaCI, pH 7.4 buffer were combined and heated for 5 min at 75 °C. The mixture was left to cool at room temperature for an hour. The cooled mixture was immediately coated on polystyrene microtiter wells (Nunc) in a concentration of 1 or 2 µg per ml phosphate-buffered saline (PBS, pH 7.4). Each well received 100 µl of coating solution.

### Example 2

### Assays for anti-DNA-antibody detection

1. Polystyrene microtiter wells were coated with the single-stranded oligonucleotides 2667 or 2668 or with the duplex DNA (2667 + 2668) obtained in Example 1. The coating concentration was 1 or 2 µg per ml PBS, pH 7.4. The coating volume was 100 µl. The plates were incubated at room temperature without cover until dry.
2. The plates were washed three times with 10 mM PBS, pH 7.4, containing 0.05% Tween 20 (Fluka AG).
3. Postcoating was carried out by applying a volume of 100 µl of Dulbecco buffer containing 0.5% bovine serum albumin (BSA) per well to block the nonspecific binding. The incubation took one hour at 37°C under plastic adhesive cover to prevent the differential evaporation.
4. The plates were washed as in 2, and airdried, and stored at +4 °C for later use.

### Example 3

### The detection of autoantibodies against telomeric sequences

1. Serum from diagnosed SLE patients and healthy controls, respectively, were diluted 1:10 in Dulbecco's buffer containing 0.5% BSA and 0.05% Tween 20 and a volume of 100 µl of the serum dilution was incubated for two hours at 37°C in the coated wells under plastic adhesive cover.
2. The plates were washed three times with 10 mM PBS, pH 7.4, containing 0.05% Tween 20 (Fluka AG).
3. A volume of 100 µl of alkaline phosphatase-labeled swine anti-human IgG (for IgG class autoantibody determination, Orion Diagnostica, Espoo, Finland) or alkaline phosphatase-labeled swine anti-human IgM (for IgM class autoantibody determination) diluted 1:200 in Dulbecco's buffer containing 0.5% BSA and 0.05% Tween 20.
   The incubation took place for one hour at 37°C.
4. The plates were washed as in 2.
5. A volume of 100 µl of substrate, 0.2% para-nitrophenyl phosphate disodium salt in diethanolamine buffer, pH 10.0, was added per well, and the plates were incubated for 30 min at room temperature.
6. A volume of 100 µl of 1 M sodium hydroxide (NaOH) was added per well to stop the reaction.
7. The absorbances of the wells were recorded using a Titertek Multiscan spectrophotometer using the wavelength 405 nm.

The results of the test are shown in Tables 1 and 2.

**TABLE 1:**

| SLE-patient assays | | | | | | |
|---|---|---|---|---|---|---|
| | IgG-class autoantibody | | | IgM-class autoantibody | | |
| Patient | 2667 | 2668 | 2667 + 2668 | 2667 | 2668 | 2667 + 2668 |
| 1. | 0.158 | 0.524 | 0.166 | 1.134 | 1.132 | 1.118 |
| | 0.152 | 0.449 | 0.093 | 1.167 | 1.132 | 1.119 |
| | | | | | | |
| 2. | 0.095 | 0.268 | 0.132 | 0.017 | 0.782 | 0.119 |
| | 0.095 | 0.237 | 0.119 | 0.012 | 0.700 | 0.110 |
| | | | | | | |
| 3. | 0.278 | 1.456 | 0.661 | 0.055 | 0.225 | 0.090 |
| | 0.308 | 1.299 | 0.584 | 0.051 | 0.228 | 0.092 |
| | | | | | | |
| 4. | 0.434 | 0.699 | 0.185 | 0.058 | 0.200 | 0.113 |
| | 0.419 | 0.700 | 0.165 | 0.057 | 0.190 | 0.106 |
| | | | | | | |
| 5. | 0.173 | 0.442 | 0.223 | 0.215 | 1.033 | 0.351 |
| | 0.201 | 0.393 | 0.217 | 0.201 | 1.092 | 0.352 |
| | | | | | | |
| 6. | 0.077 | 0.833 | 0.276 | 0.045 | 0.330 | 0.161 |
| | 0.088 | 0.615 | 0.248 | 0.041 | 0.299 | 0.160 |
| | | | | | | |
| 7. | 0.257 | 0.201 | 0.186 | 0.034 | 0.189 | 0.048 |
| | 0.264 | 0.208 | 0.156 | 0.035 | 0.189 | 0.048 |
| | | | | | | |
| 8. | 0.090 | 0.058 | 0.042 | 0.039 | 0.116 | 0.037 |
| | 0.106 | 0.083 | 0.030 | 0.040 | 0.093 | 0.033 |
| | | | | | | |
| 9. | 0.559 | 1.082 | 0.708 | 0.030 | 0.244 | 0.064 |
| | 0.639 | 0.991 | 0.632 | 0.026 | 0.209 | 0.061 |
| | | | | | | |
| 10. | 0.093 | 0.069 | 0.035 | 0.038 | 0.253 | 0.079 |
| | 0.104 | 0.065 | 0.029 | 0.029 | 0.225 | 0.074 |
| | | | | | | |
| 11. | 0.033 | 0.120 | 0.034 | 0.211 | 0.731 | 0.376 |
| | 0.022 | 0.090 | 0.036 | 0.233 | 0.802 | 0.383 |
| | | | | | | |
| 12. | 0.359 | 0.908 | 0.284 | 0.020 | 0.219 | 0.083 |
| | 0.394 | 0.852 | 0.262 | 0.038 | 0.251 | 0.079 |
| | | | | | | |
| 13. | 0.047 | 0.136 | 0.038 | 0.038 | 0.507 | 0.122 |
| | 0.031 | 0.076 | 0.032 | 0.123 | 0.536 | 0.129 |
| | | | | | | |
| 14. | 0.061 | 0.184 | 0.059 | 0.163 | 1.317 | 0.396 |
| | 0.046 | 0.137 | 0.054 | 0.168 | 1.573 | 0.416 |
| | | | | | | |
| 15. | 0.822 | 1.918 | 1.154 | 0.039 | 0.116 | 0.037 |
| | | | | 0.040 | 0.093 | 0.033 |
| | | | | | | |
| 16. | 0.086 | 0.628 | 0.073 | | | |
| | | | | | | |
| 17. | 1.794 | 1.832 | 1.597 | | | |

**TABLE 2:**

| Control assays (no SLE-diagnosis) | | | | | | |
|---|---|---|---|---|---|---|
| | | IgG-class autoantibody | | IgM-class autoantibody | | |
| Patient | 2667 | 2668 | 2667 + 2668 | 2667 | 2668 | 2667 + 2668 |
| A. | 0 | 0.001 | 0.004 | 0.014 | 0.146 | 0.058* |
| | | | | | | |
| B. | 0.024 | 0.005 | 0.002 | 0.006 | 0.056 | 0.023* |
| | 0.001 | 0.018 | 0.004 | 0.016 | 0.074 | 0.039* |
| | | | | | | |
| C. | 0 | 0 | 0 | 0.005 | 0.121 | 0.058** |
| | 0 | 0 | 0 | 0.021 | 0.165 | 0.078** |
| | | | | | | |
| D. | 0.002 | 0.002 | 0.005 | 0 | 0.042 | 0.025* |
| | 0 | 0 | 0.002 | 0.006 | 0.060 | 0.047* |
| | | | | | | |
| E. | 0.002 | 0 | 0 | 0 | 0 | 0.008* |
| | 0 | 0 | 0.002 | 0.009 | 0.025 | 0.018* |
| | | | | | | |
| F. | 0.001 | 0.003 | 0 | 0 | 0.050 | 0.029* |
| | 0.002 | 0 | 0 | 0.003 | 0.082 | 0.033* |
| | | | | | | |
| G. | 0.005 | 0.003 | 0.002 | 0 | 0 | 0.004 |
| | 0.005 | 0 | 0.001 | 0.004 | 0.005 | 0.010 |

| | | | | | | |
|---|---|---|---|---|---|---|
| Note: 2667 and 2668 comprise single-stranded telomeric DNA 2667 + 2668 comprises double-stranded DNA * Patients with suspected viral infection from the diagnostic routine | | | | | | |
| ** Patients C was born in 1992 | | | | | | |

The above results clearly show that the serum of SLE patients demonstrate a high level of anti-DNA-antibodies binding to the telomeric sequences while normal control individuals have a significantly lower level, almost negligible in the IgG class. The present assay can thus be used for screening SLE.

### Example 4

### Inhibition of anti-DNA-antibody binding to immobilized telomeric sequences

To provide proof that the binding is specific and that the antibodies can be neutralized with the specific telomeric sequence, the assay was performed as described above in Example 3 with the following modification. An amount of 5 µg/ml of oligonucleotide 2667, 2668 or the duplex DNA (2667 + 2668), respectively, was added to a 1:25 dilution of patient serum and incubated for one hour at room temperature. After this incubation a volume of 100 µl of each incubation was added per well coated with the identical telomeric sequence (as in Example 2). Incubation lasted for 2 hours at 37 °C, and the IgG or IgM class autoantibody binding was assayed using the corresponding labeled swine anti-IgG or anti-IgM antibodies, respectively, as in Example 3. The same patient serum diluted 1:25 without an addition of telomeric sequence served as a control.

The results are shown in Table 3.

**TABLE 3**

| | | | | |
|---|---|---|---|---|
| Inhibition of IgG class autoantibody binding to immobilized telomeric sequences by the telomeric sequence indicated in the Table. | | | | |

| | Immobilized sequence 2667 | | | |
|---|---|---|---|---|
| SLE No. | without inhibitor | inh. 2667 | inh. 2668 | inh. 2667+2668 |
| 1. | 1.840 | 0.037 | 0.151 | 0.035 |
| | 1.736 | 0.036 | 0.201 | 0.0221 |
| | | | | |
| 2. | 0.320 | 0.042 | 0.030 | 0.009 |
| | 0.300 | 0.045 | 0.048 | 0.029 |
| | | | | |

| | Immobilized sequence 2668 | | | |
|---|---|---|---|---|
| | without inhibitor | inh. 2667 | inh. 2668 | inh. 2667+2668 |
| 1. | 1.131 | 0.186 | 0.036 | 0.077 |
| | 0.963 | 0.101 | 0.022 | 0.075 |
| | | | | |
| 2. | 0.829 | 0.027 | 0.031 | 0.064 |
| | 1.035 | 0.034 | 0 | 0.039 |

| | Immobilized sequence 2667 + 2668 | | | |
|---|---|---|---|---|
| | without inhibitor | inh. 2667 | inh. 2668 | inh. 2667 + 2668 |
| 1. | 1.047 | 0.179 | 0.133 | 0.098 |
| | 1.134 | 0.163 | 0.142 | 0.070 |
| | | | | |
| 2. | 0.223 | 0.042 | 0.027 | 0.031 |
| | 0.270 | 0.045 | 0.040 | 0.031 |

Inhibition of IgM class autoantibody binding to immobilized telomeric sequences by the telomeric sequences indicated in the Table.

| | Immobilized sequence 2667 | | | |
|---|---|---|---|---|
| SLE No. | without inhibitor | inh. 2667 | inh. 2668 | inh. 2667+ 2668 |
| 1. | 0.244 | 0.078 | 0.046 | 0.029 |
| | 0.214 | 0.061 | 0.029 | 0.051 |
| | | | | |
| 2. | 0.119 | 0.029 | 0.018 | 0.032 |
| | 0.153 | 0.039 | 0.100 | 0.053 |

| | Immobilized sequence 2668 | | | |
|---|---|---|---|---|
| | without inhibitor | inh. 2667 | inh. 2668 | inh. 2667 + 2668 |
| 3. | 0.487 | 0.119 | 0.071 | 0.170 |
| | 0.489 | 0.139 | 0.011 | 0.125 |
| | | | | |
| 4. | 0.289 | 0.053 | 0 | 0.073 |
| | 0.278 | 0.147 | 0 | 0.073 |

| | Immobilized sequence 2667 + 2668 | | | |
|---|---|---|---|---|
| | without inhibitor | inh. 2667 | inh. 2668 | inh. 2667 + 2668 |
| 1. | 0.355 | 0.101 | 0.059 | 0.078 |
| | 0.292 | 0.106 | 0.049 | 0.097 |
| | | | | |
| 2. | 0.074 | 0.121 | 0.020 | 0.012 |
| | 0.075 | 0.068 | 0.064 | 0.060 |

The above test results show that the telomeric sequences are capable of binding to anti-DNA-antibodies present in serum in vitro. This clearly implies that said telomeric sequences are capable of inhibiting the telomere-specific anti-DNA-antibody binding site also in vivo. The telomeric sequences are therefore proposed for being used as specific therapeutic agents in patients suffering from autoimmune diseases which include the presence of anti-DNA-antibodies in the body fluid of said patient.

### Example 5

### Inhibition of IgG class autoantibody binding to parts of the telomeric sequence

The test of Example 4 was repeated for the partial sequences GGG and CCC, respectively. The results are shown in the Table below.

| | Immobilized sequence 2667 | | |
|---|---|---|---|
| Patient no. | without inhibitor | inh. GGG | inh. CCC |
| 3. | 0.600 | 0.528 | 0.245 |
| | | | |
| 4. | 0.327 | 0.281 | 0.198 |

| | Immobilized sequence 2668 | | |
|---|---|---|---|
| Patient no. | without inhibitor | inh. GGG | inh. CCC |
| 3. | 0.765 | 0.734 | 0.528 |
| | | | |
| 4. | 0.943 | 0.875 | 0.807 |

| | Immobilized sequence 2667 + 2668 | | |
|---|---|---|---|
| Patient no. | without inhibitor | inh. GGG | inh. CCC |
| 3. | 0.372 | 0.323 | 0.323 |
| | | | |
| 4. | 0.147 | 0.121 | 0.143 |

The results indicate that some binding also takes place to parts of the telomeric sequence.

### Example 6

### Immobilization of streptavidin-biotinylated telomeric sequences

### 1. Preparation of streptavidin-coated plates, strips, Latex particles and filter paper

A volume of 100 µl of purified streptavidin (1.25-10 µg/ml), purchased from Algol Oy, Finland, was immobilized in 0.01 M PBS (phosphate-buffered saline), pH 7.4, at room temperature. The unbound streptavidin was removed by three washes with the washing buffer 10 mM PBS containing 0.15 M NaCI and 0.05% Tween 20 (Fluka AG, Germany), air-dried and stored at +4 °C, if not immediately used. A coating concentration of 2 µg/ml was later used for the biotinylated oligonucleotide binding.

### 2. Synthetic telomeric sequences

Biotinylated synthetic oligonucleotides were purchased from Pharmacia, Sweden. The following oligonucleotides were synthetized:

EMS 1 + 2 and SME 1+2: Duplex DNAs (EMS 1 + 2 and SME 1 + 2) were prepared in the laboratory as follows:

A volume of 50 µl (50 µg) of each oligo (EMS 1 and EMS 2, and SME 1 and SME 2) in 10 mM Tris-1 mM EDTA containing 50 mM NaCI, pH 7.4, buffer were combined and heated for 5 min at 75 °C. The mixture (EMS 1+2, or SME 1+2) was left to cool at room temperature for an hour. The cooled mixture (100 µl) was immediately diluted (200 ng/ml) in a binding buffer 0.01 M PBS containing 0.15 NaCI, 0.05% Tween 20 and 0.5% bovine serum albumin (BSA) and let to bind the immobilized streptavidin for 2 h at 37 °C. The same binding (or incubation) buffer was used in complex formation of streptavidin-EMS 1 (or -SME 1) and streptavidin-EMS 2 (or -SME 2) complexes.

The solid-phase bound streptavidin-oligonucleotide complexes, after three washes with the above washing buffer, were used for anti-DNA-antibody detection. The solid-phase complexes were stored for two months at room temperature, +4 °C and -20 °C without statistically significant loss of binding activity.

The concentration of oligonucleotides worked well in the range of 7-500 ng/ml, but the concentration of 200 ng/ml was selected for anti-DNA-antibody detection. Maximum binding was obtained already in 30 min at + 37 °C.

### Example 7

### Detection of autoantibodies against telomeric sequences

1. Serum (and cerebrospinal fluid) from diagnosed systemic lupus erythematosus (SLE), rheumatoid arthritis and psoriasis patients as well as healthy controls, respectively, were diluted 1:50 in the binding buffer of Example 6 containing 0.5% BSA and 0.05% Tween 20. A volume of 100 µl of the serum dilution was applied per well containing streptavidin-telomere complexes, and incubated for one hour at +37°C (or at room temperature) under plastic adhesive cover.
2. The wells were washed three times with 10 mM PBS, pH 7.4, containing 0.05% Tween 20.
3. A volume of 100 µl of alkaline phosphatase-labeled swine anti-human IgG (gamma-chain specific), IgM (µ-chain specific) or IgA (alfa-chain specific) conjugate (Orion Diagnostica, Espoo, Finland) diluted 1:200 in the 10 mM PBS, pH 7.4 containing 0.5% BSA and 0.05% Tween 20.
   The incubation took place for one hour at 37 °C.
4. The wells were washed as in 2.
5. A volume of 100 µl of substrate, 0.2% para-nitrophenyl phosphate disodium salt in diethanolamine buffer was added per well, and the plates were incubated for 30 min at room temperature.
6. A volume of 100 µl of 1 M sodium hydroxide (NaOH) was added per well to stop the reaction.
7. The absorbances of the wells were recorded using a Titertek Multiskan spectrophotometer (Flow Laboratories, U.K.) using the wavelength 405 nm.

Some of the results of the test are indicated in Tables 4, 5, 6 and 7, which show mean values of representative ones of the performed duplicate tests. In the Tables W stands for woman and M stands for male, the figures in connection therewith indicating the patients age.

The Tables show that SLE and RA patients have an elevated level of anti-DNA-antibodies compared to the healthy controls. Some psoriasis patients also have elevated levels of anti-DNA-antibodies. The healthy controls had clearly lower absorbance levels. The cut-off value the absorbance at 405 nm for the assay of the present test is 0.150 (A 405 = 0.150). Levels elevated above 0.150 absorbance were obtained primarily in the IgG class in SLE patient sera and in the IgM class in RA patient sera.

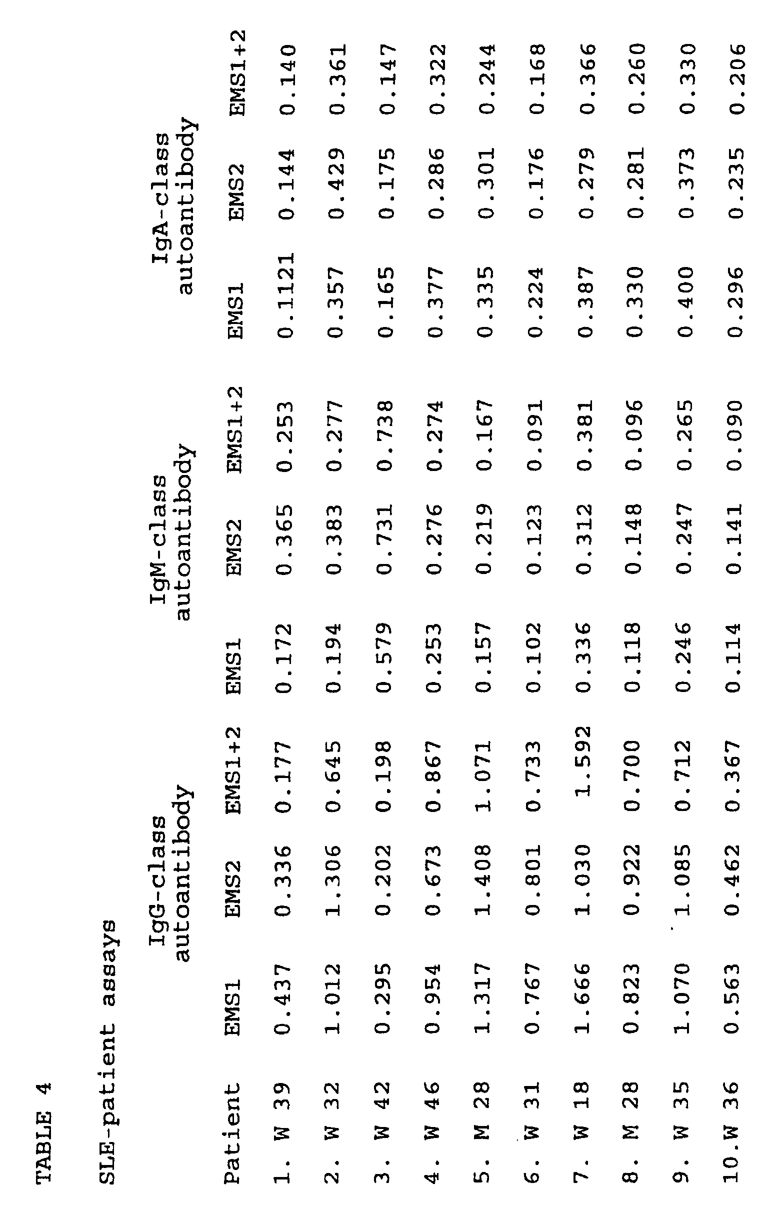

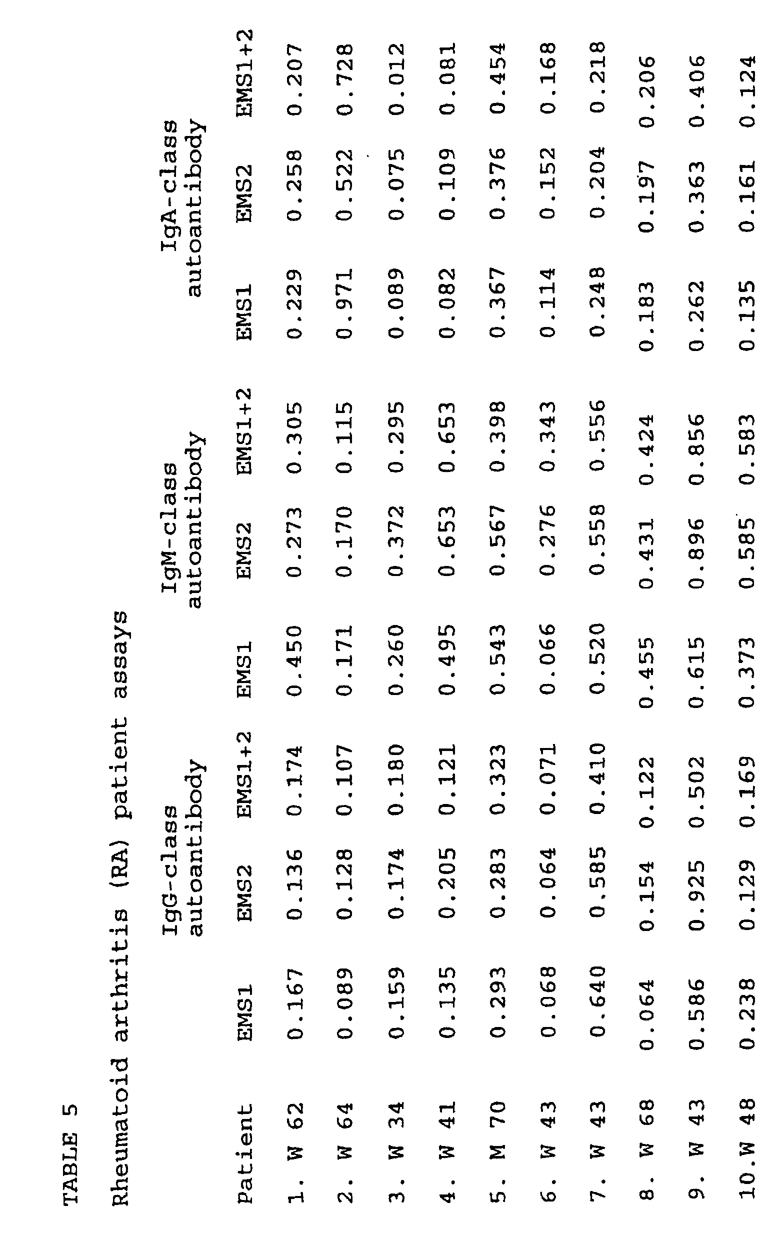

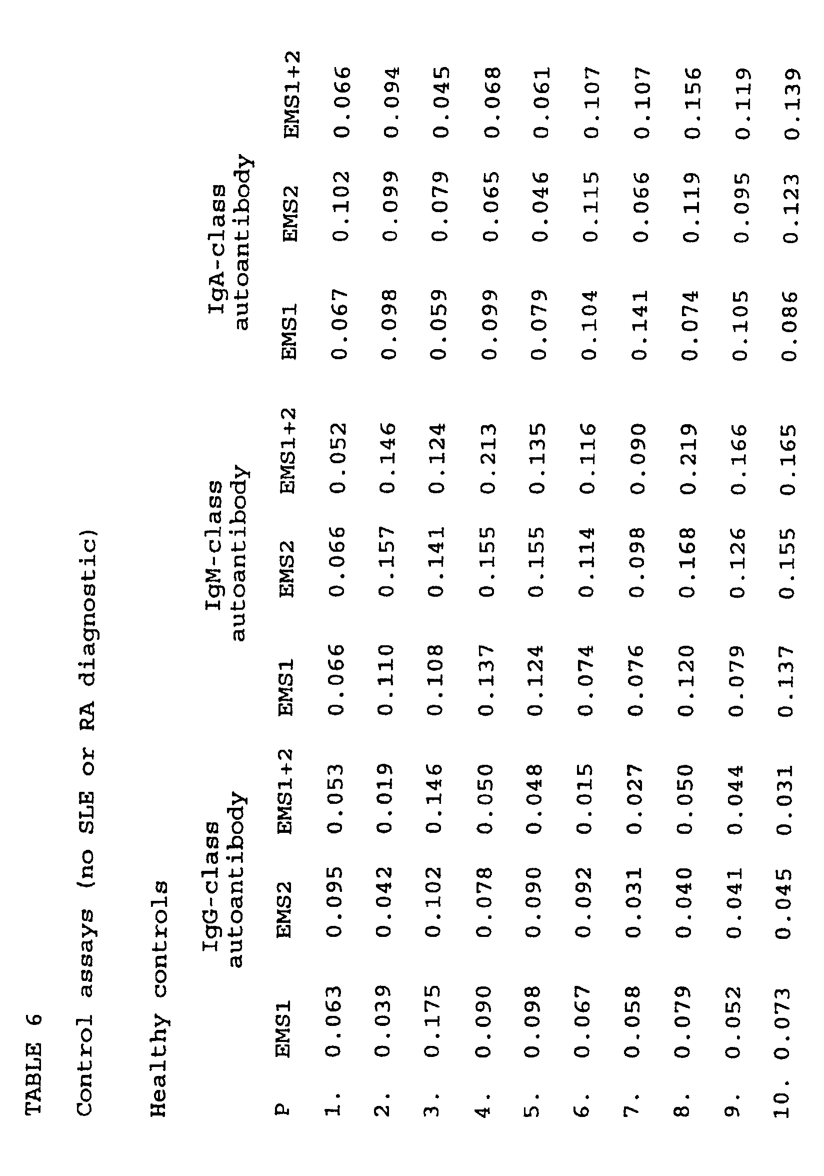

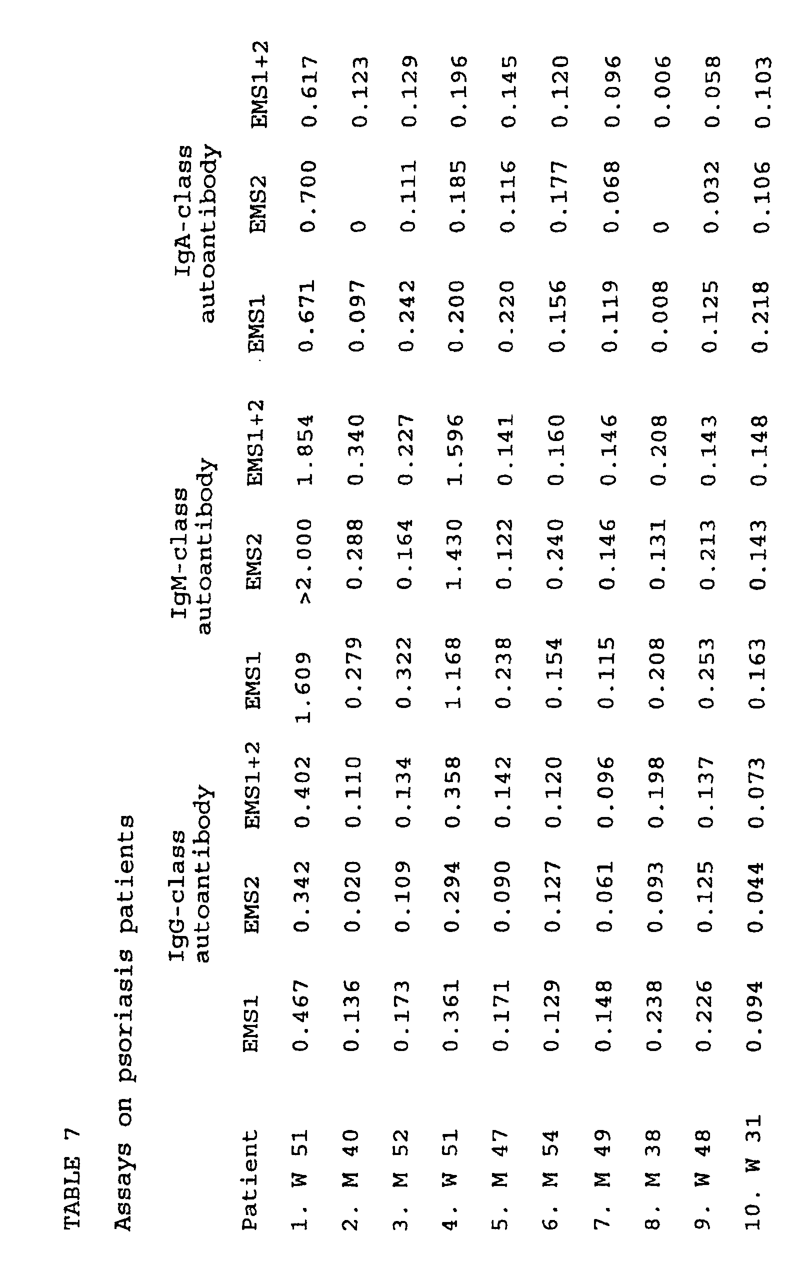

### Example 8

### Latex agglutination test

1. A volume of 50 µl of polystyrene Latex beads (Sigma, St. Louis, U.S.A) with a particle diameter 1.1 µm (µ) was incubated in 10 mM PBS, pH 7.4 at a concentration of 5 µg streptavidin/ml, overnight at room temperature.
2. Any unbound streptavidin was removed from the bound by washing with 0.01 M PBS, pH 7.4, followed by centrifugation.
3. A volume of 50 µl of biotinylated oligonucleotide EMS 1 of Example B (5 µg/ml in mM PBS, pH 7.4, containing 0.05% Tween and 0.5% BSA) was incubated with streptavidin-coated Latex particles.
4. Any unbound oligonucleotides were removed from the bound by washing with 0.01 M PBS, pH 7.4, followed by centrifugation.
5. Sera from SLE patients ware diluted 1:5 with incubation buffer (glycine 7.5 g/l supplemented with NaCl 10 g/l, pH 8.2).
6. A drop (50 µl) of patient serum dilution was incubated with a drop of coated Latex particles.
7. With anti-DNA positive sera a visible agglutination of Latex particles occurred, whereas the negative sera gave no reaction.

The test provides a rapid qualitative test for the diagnosis of SLE.

### Example 9

### Complement fixation test

1. Anti-DNA-antibody positive SLE sera and control sara, respectively, were serially diluted in veronal buffer (also called barbital buffer), pH 7.6. A volume of 25 µl of diluted specimens were transferred to the U-shaped microtiter wells.
2. The unlabeled telomeric sequence, 2667, 2668 or 2667 + 2668, respectively, as defined in Example 1 was added into the serum dilution at a concentration of 10µg/ml, and a volume of 25 µl was added per sample well.
3. Guinea pig complement (25 µl) was added per well overnight.
4. In the morning a haemolytic system (25 µl of sheep red cell suspension and 25 µl of rabbit anti-sheep antibodies) was applied into the sample wells. The reaction mixtures were shaken and then incubated for 30 min at 37 °C, shaken again and reincubated for 30 min at 37 °C.
5. The results were visualized both objectively and by the inhibition of a light path through the reaction mixture, as measured with a spectrophotometer. A total haemolysis (the disruption of cells) was obtained with sera without antibodies to the antigen (in this case the telomeric sequences) tested. The presence of anti-DNA-antibodies in the serum specimens tested resulted in a visible red pellet in the corresponding reaction well (based on the consumption of the complement in the reaction system).

The complement fixation test provides a rapid qualitative test for the diagnosis of anti-DNA-antibodies in a sample.

### Example 10

### Detection of anti-telomere antibodies using immunofluorescence antibody technique

1. The metaphase chromosomes of three day-cultures of blood lymphocytes, fixed in glass slides, were obtained from the Department of Obstetrics and Gynaecology, Helsinki University Central Hospital.
2. Serum samples with and without anti-DNA antibodies, respectively, were diluted 1:20 in 0.01 M PBS, pH 7.4, containing 0.5% BSA (bovine serum albumin) and incubated on the slides in a humid chamber for 30 min at + 37 °C.
3. The slides were washed three times for 5 min at room temperature, and air-dried.
4. The slides were incubated with 1:10 dilution of FITC (fluorescein isothiocyanate)-conjugated goat anti-human IgG antibodies (Kallestad, Austin, Texas 78746, USA) for 30 min in a humid chamber at + 37 °C.
5. The slides were washed as in 3) and air-dried.
6. A mounting medium and cover slides were added and the slides were ready for microscopic examination.

### Results:

The anti-DNA sera gave a positive speckled-like fluorescent staining (a typical telomere staining) with 1:50 magnification and oil, whereas with anti-DNA negative sera no staining was seen.

The procedure provides a rapid test for detecting anti-DNA-antibodies in a sample.

### Example 11

### Rapid detection of anti-DNA-antibodies by labeled anti-Ig

1. A few drops of a telomeric sequence according to Example 1 and Example 6, respectively, are added to pieces of filter paper.
2. A drop of patient serum or a drop of spit (saliva), respectively, is added onto the drops of telomere.
3. The filters are rinsed to remove any unbound fluid.
4. A drop of labeled anti-human IgG or labeled anti-human IgA, respectively, is added to the filters at the previous drop sites.
5. In case of the presence of anti-DNA-antibodies in the sample drops, the label gives a visible positive reading when using a color-forming or precipitating substrate.

The above test provides a crude but a very cheap, simple and quick detection of anti-DNA-antibodies in body fluids without the need for any special equipment.

### Example 12

### Use of differential antibody avidity (affinity) in single- or double-stranded DNA binding

Four patient sera of typical SLE cases were selected for the test. The patients were 16 (P16), 17 (P17), 38 (P38) and 63 (P63) years old. The test was performed as described in Example 7, in parallel with the otherwise similar experiment, with the exception that after the serum incubation the bound DNA antibodies were washed three times for 5 min at room temperature with freshly prepared 8 M urea diluted in 10 mM PBS, pH 7.4, containing 0.05% Tween 20. This treatment is known to release the low avidity antibodies from the antigen but has no or only a minor effect on the high avidity antibodies.

The results showed that the anti-DNA antibodies had a different and partly age-related affinity for telomeres. Three of the four patients showed a low avidity anti-DNA binding to the single-stranded DNA (EMS 1 or EMS 2), since the bound antibodies (P16, P38 and P63) could be removed with 8 M urea. The avidity of antibodies to the duplex DNA (EMS 1 + 2) was higher, since only in two cases the bound anti-DNA-antibodies (P38 and P63) were totally removed with 8 M urea.

The test can be used to indicate the length of time that the patient has been exposed to anti-DNA-antibodies. A prolonged disease and a consequent long exposure time leads to anti-DNA-antibodies having a higher affinity to the telomeric sequence. The age of the patient should generally also be taken into account, however, since the amount of telomeres is known to be related to the age of a person.

### Example 13

### Storage of coated streptavidin-telomere complexes at different temperatures

The capacity (or activity) of streptavidin-telomere complexes to bind anti-DNA-antibodies from the patient serum was tested after the plates coated with streptavidin-telomers (EMS 1, EMS 2 or EMS 1+2 of Example 6) were incubated at +37°C, at room temperature, at + 4°C or at -20°C for one day (the baseline), two weeks, one month or two months. In each case the test was performed using the same patient serum (1:50 dilution). the same conjugate (1:200 dilution of alkaline phosphatase labelled swine anti-human IgG) and the same buffers and reagents as described in Example 7 for the detection of autoantibodies against telomeric sequences. Each measurement was performed in triplicate wells.

The results show that no binding activity of the coatad complexes was lost during the storage at +4°C or -20°C in two months. A 20% decrease in the absorbance value (directly proportional to the binding activity of coated complexes) was obtained after storage at room temperature, and a 30% decrease at +37°C, compared to the baseline value.

### Example 14 a

### Anti-DNA-antibody separation column using telomere affinity column

1. Plastic disposable columns with a 4 µm (µ) filter plug were packed with suspension of polystyrene Latex particles (Serva, Heidelberg, Germany) with a particle size 1.1 µm (µ).
2. Purified streptavidin et a concentration of 2 µg/ml PBS, pH 7.4, was added into the column, and incubated with the suspension in a roller overnight at room temperature.
3. Any unbound streptavidin was removed by centrifugation, and the streptavidin-coated particles were washed ten times with PBS, pH 7.4.
4. The biotinylated telomeric sequences EMS 1, EMS 2 or EMS 1+2 of Example 6 at a concentration of 2 µg/ml were incubated with the streptavidin-coated Latex particles for 2 hour in a roller at room temperature.
5. Any unbound telomeric sequences were removed by washing the column ten times with PBS.
6. The anti-DNA sera of patients with SLE were applied on the column, incubated for 5 min in the column at room temperature, and then passed through the column.
7. The column was washed ten times with PBS, pH 7.4, and the telomeric sequence-bound anti-DNA-antibodies were eluted using a 5 min repetitive incubation of 0.1 M glycine, pH 3, in a roller, followed by centrifugation. A volume of 200 µl per fraction after each incubation was collected, followed by adjustment of the pH to 7.4.
8. The results clearly show that anti-DNA-antibodies bind to the telomere affinity column. With use of small columns (0.4 ml of affinity matrix) and undiluted anti-DNA-antibody specimens, about a half of the antibodies originally present in the patient serum (A405 0.963-1.420) were bound to the column (A405 0.444-0.670). With an increase of the column size or by recycling the column, all anti-DNA-antibodies that are specific to telomeric sequences are bound, and will be removed from the sera.

### Example 14 b

### Anti-DNA-antibody removal in batch separation

The removal of anti-DNA-antibodies from the sera of Example 13a was carried out also by the use of a batch separation system, where the affinity column was omitted. In this case the affinity matrix was incubated with the anti-DNA sera, followed by centrifugation and/or direct filtration of the serum through the 0.22 µm filter column.

Removal of anti-DNA-antibodies was successfully carried out also in this way.

### Example 15

### Toxicity test of telomeric sequences in vivo

Each rabbit (8 weeks old) received intravenously 100 µg of the sequences EMS 1, EMS 2 or EMS 1 + 2 of Example 6 in complete Freund's adjuvant three times at ten days' intervals. The rabbits were examined daily for two months for their physical status, which was found to be normal.

The detection of antibodies against telomeric sequences was performed similarly as in humans, except for the conjugate which was alkaline phosphatase-labeled swine anti rabbit IgG. No antibodies against telomeric sequences were detected in the sera of the rabbits.

### Example 16

### Purified human F(ab')₂ or Fab antibodies to telomeric sequences as therapeutic agents

### Purification of human IgG from a patient serum containing anti-DNA-antibodies

1. Human IgG from a serum of a patient with SLE was purified using a ProSep^{RA} high capacity matrix (Bioprocessing, Co Durham, England), which consists of protein A-coated glass beads with a binding capacity of 40 mg IgG/ml beads.
   This method can also be used to absorb the IgG-class anti-DNA-antibodies from patient serum.
2. The serum was passed through the column, and the column washed until A280 = 0 in the flow-through.
3. The bound IgG was eluted from the column with 0.1 M glycine, pH 3.0. The fractions containing IgG were pooled, neutralized with 1 M Tris buffer, dialyzed against PBS, pH 7.4, overnight, and measured for the protein content.

### Purification of F(ab')₂ antibodies from purified IgG

1. In the purification of F(ab')₂ fragments from the purified IgG, the Immuno PureR F(ab')₂ Preparation kit (Pierce, no. 44888, Rockford, Illinois 61105, USA) was used according to manufacturer's instructions. In principle, the crude pepsin-IgG digest is passed through the protein A-column, where the whole IgG and the Fc part of IgG are bound, but not the F(ab')₂ fragments, which elute from the column, are pooled and dialyzed against PBS, pH 7.4.

### Result:

The purified F(ab')₂ antibodies bound the immobilized telomeric sequences in a dose-dependent fashion, as detected in EIA using alkaline phosphatase-conjugated goat anti-human IgG, F(ab')₂, (Pierce, Rockford, Illinois, 61105, USA) for the telomere-bound F(ab')₂ antibody detection. The result shows that F(ab')₂ antibodies bind telomeric sequences. They can be purified to the monospecificity using a telomere affinity chromatography. Being of human origin they can be safely used intravenously as competitors for autologous IgG-class anti-DNA antibodies for binding telomeric sequences. The F(ab')₂ antibodies lack effector functions and do not activate the complement cascade when bound to an antigen.

The treatment of F(ab')₂ antibodies with reducing agents results in the formation of monomeric Fab antibodies, which are the biological form of the recombinant Fabs prepared using phage display systems. The monomeric Fabs, whether of biological origin or preferably produced by recombinant DNA technology, may be used intravenously as therapeutic agents the same way as the F(ab')₂ for competing with and thus preventing the destructive action of whole anti-DNA-antibodies in patient body fluids.

### Example 17

### The subclasses of the IgG-class antibodies recognized by telomeric sequences

The test was performed as described in Example 7, with the exception that alkaline-phosphatase-labeled mouse anti-IgG1, IgG2, IgG3 or IgG4 antibodies (Zymed Laboratories, Inc, Calif. USA) were used to detect the subclass-specificity of IgG anti-DNA-antibody.

The results of 20 tested sera from patients with SLE show that the anti-DNA autoantibodies recognized by telomeric sequences EMS1, EMS2 or EMS1 +2 are primarily IgG1-class and to some extent IgG2-class antibodies.

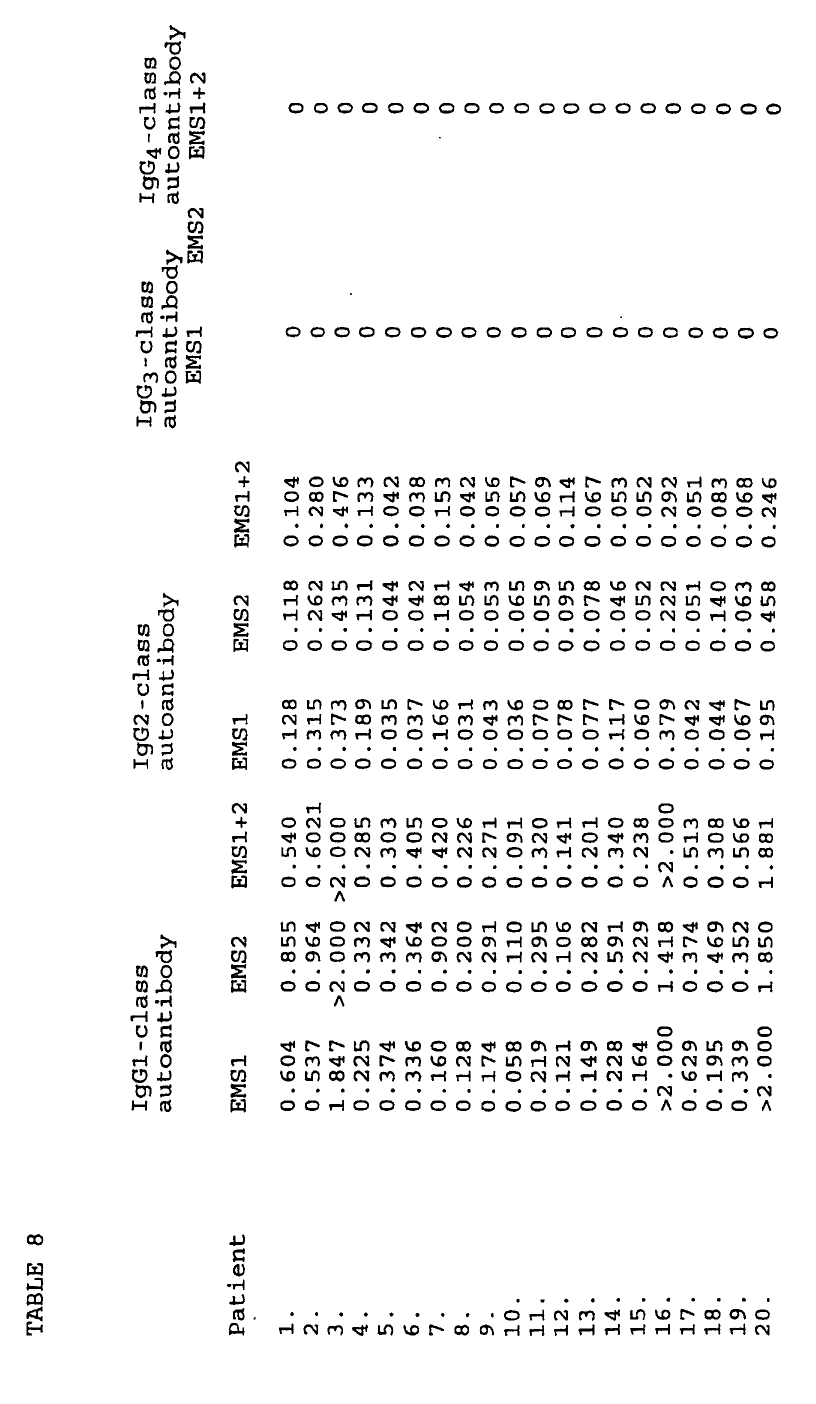

### Example 18

### Pharmaceutical preparation

A pharmaceutical preparation is prepared from the telomeric sequence 2667: 5'-TTAGGG TTAGGG TTAGGG TTAGGG TTAGGG-3' by dissolving 1 µg/ml of said sequence in physiological buffer. The solution is stored at 4 °C under sterile conditions.

A second pharnmaceutical preparation is prepared in the corresponding way from the IgG class Fab antibodies of Example 16.

### Example 19

### Administering a telomeric sequence or Fab to SLE patients

The serum from SLE patients is diagnosed with an assay according to the invention. Said serum is found to contain an elevated amount of anti-DNA-antibodies of the IgG class binding predominantly to the telomeric sequence 2667 of Example 1.

The pharmaceutical preparations of Example 18 are administered to said patients intravenously at a dose of 100 ml three times in three weeks. After the administration period the serum of said patients is again tested in an assay according to the invention and the absorbance levels are found to be reduced from the original ones due to a binding of the telomeres or Fabs, respectively, to anti-DNA-antibodies circulating in the blood-stream.

## Claims

1. A method for detecting anti-DNA-autoantibodies in a sample, **characterized in** contacting said sample with a telomeric sequence of DNA selected from a group consisting of a single-stranded telomeric sequence, a complementary strand thereto, a double-stranded telomere, and a part or a repeat or a combination of any of the foregoing, to provide binding of said autoantibodies to said telomeric sequence of DNA.

2. The method according to claim 1, wherein the telomeric sequence is selected from the group consisting of the vertebrate telomere strand 5'-TTAGGG-3', the complementary strand thereto, a double-stranded vertebrate telomere, a functional part of any of said strands, a repeat or any of said strands and a combination of any of the foregoing.

3. The method according to claim 2, wherein said repeat of said telomere strand is selected from the group consisting of and a duplex thereof.

4. The method of claim 1, wherein an antibody to said telomeric sequence or a fragment of said antibody, especially a F(ab')₂ or a Fab fragment, is used in said detection.

5. The method in claim 4, wherein the immunoglobulin (Ig) class of said anti-DNA-autoantibodies is determined with the aid of anti-Ig-antibodies, especially anti-IgG, anti-IgM or anti-IgA antibodies.

6. The method of claim 5, wherein the immunoglobulin subclass of said anti-DNA-autoantibodies is determined with the aid of specific subclass anti-Ig-antibodies, especially anti-IgG1 or anti-lgG2 antibodies.

7. The method according to claim 1, wherein said sample is vertebrate body fluid.

8. The method according to claim 7, wherein the body fluid is human serum or cerebrospinal fluid.

9. The method according to claim 7 or 8 for diagnosing autoimmune diseases in vertebrates, wherein the body fluid sample from said vertebrate is contacted with the said vertebrate telomeric sequence of DNA, an elevated level of anti-DNA-autoantibodies present in said body fluid being taken as indicative of such disease in said vertebrate.

10. The method of claim 9, wherein said autoimmune disease is selected from the group consisting of Lupus Erythematosus, Rheumatoid Arthritis and Scleroderma.

11. The method of claim 10, wherein said autoimmune disease is Systemic Lupus Erythematosus (SLE).

12. The method according to claim 9, wherein said telomeric sequence is selected from the group consisting of the vertebrate telomere strand 5'-TTAGGG-3', the complementary strand thereto, a double-stranded vertebrate telomere, a functional part of any of said strands, a repeat of any of said strands and a combination of any of the foregoing.

13. The method according to claim 12, wherein said repeat of said telomere strand is selected from the group consisting of and
a duplex thereof.

14. The method of claim 9, wherein an antibody or a fragment of an antibody to said telomeric sequence, especially a F(ab')₂ or a Fab fragment is used in said diagnosis.

15. The method of claim 14, wherein the immunoglobulin (Ig) class of said anti-DNA-autoantibodies is determined with the aid of anti-Ig-antibodies, especially anti-IgG, anti-IgM or anti-IgA antibodies.

16. The method according to claim 15, wherein SLE in a human patient is detected by contacting a sample of said patient's body fluid with an immobilized vertebrate telomeric sequence to bind anti-DNA-autoantibodies present in said fluid to said immobilized telomeric sequence and detecting an elevated level of bound IgG class autoantibodies among said bound anti-DNA-autoantibodies.

17. The method according to claim 15, wherein Rheumatoid Arthritis in a human patient is detected by contacting a sample of said patient's body fluid with an immobilized vertebrate telomeric sequence to bind anti-DNA-autoantibodies present in said serum to said immobilized telomeric sequence and detecting an elevated level of bound IgM dass autoantibodies among said bound anti-DNA-autoantibodies.

18. A test kit for the detection of anti-DNA-autoantibodies, especially in the diagnosis of autoimmune diseases in vertebrates, **characterized in that** said test kit includes an immobilized vertebrate telomeric sequence of DNA selected from the group consisting of a single-stranded vertebrate telomeric sequence, a complementary strand thereto, a double-stranded vertebrate telomere, a part of a repeat or a combination of any of the foregoing, capable of binding anti-DNA-autoantibodies present in a body fluid of said vertebrate, as well as a label capable of indicating the binding of or inhibition of binding of said anti-DNA-autoantibody to said sequence.

19. The test kit of claim 18, which includes an antibody or a fragment of an antibody to said telomeric sequence, especially a F(ab')₂ or a Fab fragment, for use in said detection.

20. The test kit of claim 19, which includes specific anti-Ig-antibodies, especially anti-IgG, anti-IgM or anti-IgA antibodies for use in determining the immunoglobulin (Ig) class of said anti-DNA-autoantibodies.

21. The test kit of claim 20, which includes specific subclass anti-Ig-antibodies, especially anti-IgG1 or anti-IgG2 antibodies for the detection of immunoglobulin subclasses of said anti-DNA-autoantibodies.

22. The test kit according to claim 18, wherein said autoimmune disease is selected from the group consisting of Lupus Erythematosus, Rheumatoid Arthritis and Scleroderma.

23. The test kit according to claim 22, wherein said autoimmune disease is Systemic Lupus Erythematosus (SLE).

24. The test kit according to claim 18, wherein said telomeric sequence is selected from the group consisting of the vertebrate telomere strand 5'-TTAGGG-3', the complementary strand thereto, a double-stranded vertebrate telomere, a functional part of any of said strands, a repeat of any of said strands and a combination of any of the foregoing.

25. The test kit according to claim 24, wherein said repeat of said telomere strand is selected from the group consisting of and
a duplex thereof.

26. The test kit according to claim 18, wherein said telomeric sequence is biotinylated at the 3' or the 5' end and is immobilized on a streptavidin-coated support.

27. The test kit according to claim 19, wherein said label is provided on a telomere-specific anti-Ig-antibody, on a fragment thereof or on a second telomeric sequence.

28. The test kit according to claim 19, wherein said label is selected from the group consisting of a radioactive label, an enzyme label, a fluorochrome or fluorescence label, a dye, biotin, a luminescent label, and a coloured particle such as a coloured latex particle.

29. The test kit according to claim 19, which includes an amount of one or more labeled anti-lg-antibodies selected from the group consisting of anti-IgG, anti-IgM, anti-IgA, anti-IgD, and anti-IgE antibodies or subclasses thereof.

30. The test kit according to claim 29, wherein said anti-Ig-antibody is selected from the group consisting of anti-IgG, anti-IgM, anti-IgA, anti-IgG1 and anti-lgG2 antibodies.

31. The test kit according to claim 18 or 26, wherein said telomeric sequence is immobilized on the walls of microtiter wells, on Sepharose, on glass or plastic beads or on a porous sheet.

32. A solid-phase means for the removal of anti-DNA-autoantibodies from a fluid sample, wherein said means is in the form of an affinity column packed with solid material, such as glass or plastic beads or a porous material, including an immobilized telomeric sequence of DNA selected from the group consisting of a single-stranded telomeric sequence, a complementary strand thereto, a double-stranded telomere, a part or a repeat or a combination of any of the foregoing, capable of binding anti-DNA-autoantibodies present in a fluid.

33. The solid-phase means according to claim 32, wherein said telomeric sequence is selected from the group consisting of the vertebrate telomere strand 5'-TTAGGG-3', the complementary strand thereto, a double-stranded vertebrate telomere, a functional part of any of said strands, a repeat of an) of said strands and a combination of any of the foregoing.

34. The solid-phase means according to claim 33, wherein said repeat of said telomere strand is selected from the group consisting of and
a duplex thereof.

35. The solid-phase means according to claim 32, wherein said telomeric sequence is biotinylated at the 3' or the 5' end and is immobilized on a streptavidin-coated support

36. Use of telomeric sequences or fragments of antibodies thereto, said telomeric sequence being selected from the group consisting of a single-stranded vertebrate telomeric sequence, a complementary strand thereto, a double-stranded vertebrate telomere, and a part or a repeat or a combination of any of the foregoing, for the manufacture of a drug for the treatment of autoimmune disorders and/or diseases caused by or in volume the presence of anti-DNA-autoantibodies, said telomeric sequence being included in the drug with an acceptable pharmaceutical carrier.

37. The use according to claim 36, wherein said autoimmune disease is selected from the group consisting of Lupus Erythematosus, Rheumatoid Arthritis and Scleroderma.

38. A test kit for the detection of anti-DNA-autoantibodies, especially in the diagnosis of autoimmune diseases in vertebrates, **characterized in that** said test kit includes a non-immobilized vertebrate telomeric sequence of DNA selected from the group consisting of a single-stranded vertebrate telomeric sequence, a complementary strand thereto, a double-stranded vertebrate telomere, a part or a repeat or a combination of any of the foregoing, capable of binding anti-DNA-autoantibodies present in a body fluid of said vertebrate, as well as a label capable of indicating the binding of said anti-DNA-autoantibody to said sequence and means for detecting the binding.

39. A method of purifying an anti-telomere-antibody from a fluid by binding said anti-telomere-antibody in said fluid to a telomeric sequence selected from the group consisting of a single-stranded telomeric sequence, a complementary strand thereto, a double-stranded telomere, and a part or a repeat or a combination of any of the foregoing, immobilized In an affinity column, or a fragment of said anti-telomere-antibody.

## Patentansprüche

1. Verfahren zum Nachweis von anti-DNA-Autoantikörpern in einer Probe, **dadurch gekennzeichnet, dass** die Probe mit einer DNA-Telomersequenz, ausgewählt aus einer Gruppe bestehend aus einer einzelsträngigen Telomersequenz, einem dazu komplementären Strang, einem doppelsträngigen Telomer und einem Teil oder einer Wiederholung oder einer Kombination der vorher genannten, in Kontakt gebracht wird, um die Bindung der Autoantikörper an die DNA-Telomersequenz zu ermöglichen.

2. Verfahren nach Anspruch 1, wobei die Telomersequenz ausgewählt ist aus der Gruppe bestehend aus einem Vertebratentelomerstrang 5'-TTAGGG-3', dem dazu komplementären Strang, einem doppelsträngigen Vertebratentelomer, einem funktionellen Teil von einem der Stränge, einer Wiederholung einer der Stränge und einer Kombination der vorher genannten.

3. Verfahren nach Anspruch 2, wobei die Wiederholung des Telomerstranges ausgewählt ist aus der Gruppe bestehend aus und einem Duplex der beiden Stränge.

4. Verfahren nach Anspruch 1, wobei ein Antikörper gegen die Telomersequenz oder ein Fragment des Antikörpers, insbesondere ein F(ab')₂- oder ein Fab-Fragment, für den Nachweis verwendet wird.

5. Verfahren nach Anspruch 4, wobei die Immunglobulinklasse (Ig) der anti-DNA-Autoantikörper mit Hilfe von anti-Ig-Antikörpern, insbesondere anti-IgG-, anti-IgM- oder anti-lgA-Antikörpern, bestimmt wird.

6. Verfahren nach Anspruch 5, wobei die Immunglobulin-Unterklasse der anti-DNA-Autoantikörper mit Hilfe von spezifischen Unterklasse-anti-lg-Antikörpern, insbesondere anti-IgG1- oder anti-IgG2-Antikörpern, bestimmt wird.

7. Verfahren nach Anspruch 1, wobei die Probe Körperflüssigkeit eines Vertebraten ist.

8. Verfahren nach Anspruch 7, wobei die Körperflüssigkeit menschliches Serum oder cerebrospinale Flüssigkeit ist.

9. Verfahren nach Anspruch 7 oder 8 zur Diagnose von Autoimmunkrankheiten bei Vertebraten, wobei die Probe mit Körperflüssigkeit des Vertebraten mit der DNA-Telomersequenz des Vertebraten in Kontakt gebracht wird und ein erhöhter anti-DNA-Autoantikörperspiegel in der Körperflüssigkeit als Indikator einer solchen Krankheit in dem Vertebraten gewertet wird.

10. Verfahren nach Anspruch 9, wobei die Autoimmunkrankheit aus der Gruppe ausgewählt ist bestehend aus Lupus erythematodes, rheumatoider Arthritis und Sclerodermie.

11. Verfahren nach Anspruch 10, wobei die Autoimmumkrankheit systemischer Lupus erythematodes (SLE) ist.

12. Verfahren nach Anspruch 9, wobei die Telomersequenz ausgewählt ist aus der Gruppe bestehend aus dem Vertebratentelomerstrang 5'-TTAGGG-3', dem dazu komplementären Strang, einem doppelsträngigen Vertebratentelomer, einem funktionellen Teil der Stränge, einer Wiederholung von einem der Stränge und einer Kombination der vorher genannten.

13. Verfahren nach Anspruch 12, wobei die Wiederholung ausgewählt ist aus der Gruppe bestehend aus und einem Duplex der beiden Stränge.

14. Verfahren nach Anspruch 9, wobei ein Antikörper oder ein Fragment eines Antikörpers gegen die Telomersequenz, insbesondere ein F(ab')₂- oder ein Fab-Fragment, für die Diagnose eingesetzt wird.

15. Verfahren nach Anspruch 14, wobei die Immunglobulin (Ig)-Klasse der anti-DNA-Autoantikörper mit Hilfe von anti-Ig-Antikörpern, insbesondere anti-IgG-, anti-IgM- oder anti-IgA-Antikörpern, bestimmt wird.

16. Verfahren nach Anspruch 15, wobei SLE in einem menschlichen Patienten dadurch nachgewiesen wird, dass eine Probe mit Körperflüssigkeit des Patienten mit einer immobilisierten Telomersequenz eines Vertebraten in Kontakt gebracht wird, um in der Flüssigkeit befindliche anti-DNA-Autoantikörper an die immobilisierte Telomersequenz zu binden und einen erhöhten Spiegel von gebundenen IgG-Klasse-Autoantikörpern unter den gebundenen anti-DNA-Autoantikörpern nachzuweisen.

17. Verfahren nach Anspruch 15, wobei rheumatoide Arthritis in einem menschlichen Patienten dadurch nachgewiesen wird, dass eine Probe mit Körperflüssigkeit des Patienten mit einer immobilisierten Telomersequenz eines Vertebraten in Kontakt gebracht wird, um anti-DNA-Autoantikörper, die im Serum sind, an die immobilisierte Telomersequenz zu binden und einen erhöhten Spiegel von gebundenen IgM-Klasse-Autoantikörpern unter den gebundenen anti-DNA-Autoantikörpern nachzuweisen.

18. Testkit zum Nachweis von anti-DNA-Autoantikörpern, insbesondere für die Diagnose von Autoimmunkrankheiten in Vertebraten, **dadurch gekennzeichnet, dass** der Testkit eine immobilisierte DNA-Telomersequenz eines Vertebraten enthält, die ausgewählt ist aus der Gruppe bestehend aus einer einzelsträngigen Telomersequenz eines Vertebraten, einem dazu komplementären Strang, einem doppelsträngigen Telomer eines Vertebraten, einem Teil, einer Wiederholung oder einer Kombination von einem der vorher genannten, die anti-DNA-Autoantikörper in der Körperflüssigkeit von Vertebraten binden kann, und einen Marker, der die Bindung oder die Hemmung der Bindung des anti-DNA-Autoantikörpers an die Sequenz anzeigen kann.

19. Testkit nach Anspruch 18, der einen Antikörper oder ein Fragment eines Antikörpers gegen die Telomersequenz, insbesondere ein F(ab')₂- oder ein Fab-Fragment, enthält, zur Verwendung beim Nachweis.

20. Testkit nach Anspruch 19, der spezifische anti-Ig-Antikörper, insbesondere anti-IgG-, anti-lgM- oder anti-IgA-Antikörper, enthält, zur Verwendung bei der Bestimmung der Immunglobulin (Ig)-Klasse der anti-DNA-Autoantikörper

21. Testkit nach Anspruch 20, der eine spezifische Unterklasse von anti-Ig-Antikörpern, insbesondere anti-IgG1- oder anti-lgG2-Antikörper, enthält, zum Nachweis von Immunglobulin-Unterklassen der anti-DNA-Autoantikörper.

22. Testkit nach Anspruch 18, wobei die Autoimmunkrankeit ausgewählt ist aus der Gruppe bestehend aus Lupus erythematodes, rheumatoider Arthritis und Sclerodermie.

23. Testkit nach Anspruch 22, wobei die Autoimmunkrankheit systemischer Lupus erythematodes (SLE) ist.

24. Testkit nach Anspruch 18, wobei die Telomersequenz ausgewählt ist aus der Gruppe bestehend aus dem Vertebraten-Telomerstrang 5'-TTAGGG-3', dem dazu komplementären Strang, einem doppelsträngigen Vertebratentelomer, einem funktionellen Teil der Stränge, einer Wiederholung eines der Stränge und einer Kombination der vorher genannten.

25. Testkit nach Anspruch 24, wobei die Wiederholung des Telomerstranges ausgewählt ist aus der Gruppe bestehend aus und einem Duplex der beiden Stränge.

26. Testkit nach Anspruch 18, wobei die Telomersequenz am 3'- oder am 5'-Ende biotinyliert und an einem Streptavidin-beschichteten Träger immobilisiert ist.

27. Testkit nach Anspruch 19, wobei der Marker auf einem Telomer-spezifischen anti-lg-Antikörper, auf einem Fragment davon oder auf einer zweiten Telomersequenz bereitgestellt wird.

28. Testkit nach Anspruch 19, wobei der Marker ausgewählt ist aus der Gruppe bestehend aus einer radioaktiven Markierung, einer Enzymmarkierung, einem Fluorochrom oder einer fluoreszienden Markierung, einem Farbstoff, Biotin, einer lumineszierenden Markierung und gefärbten Teilchen wie zum Beispiel gefärbten Latexteilchen.

29. Testkit nach Anspruch 19, der eine Menge eines oder mehrerer markierter anti-lg-Antikörper enthält, die ausgewählt sind aus der Gruppe bestehend aus anti-IgG-, anti-IgM-, anti-lgA-, anti-lgD- und anti-lgE-Antikörpern oder Unterklassen davon.

30. Testkit nach Anspruch 29, wobei der anti-lg-Antikörper ausgewählt ist aus der Gruppe bestehend aus anti-IgG-, anti-IgM-, anti-lgA-, anti-IgG1- und anti-IgG2-Antikörpern.

31. Testkit nach Anspruch 18 oder 26, wobei die Telomersequenz an den Wänden von Mikrotiterplattenvertiefungen, an Sepharose, an Glas- oder an Plastikperlen oder an einer porösen Unterlage immobilisiert ist.

32. Festphasenmittel für das Entfernen von anti-DNA-Autoantikörpern aus einer flüssigen Probe, wobei das Mittel in Form einer Affinitätssäule vorliegt, die mit Festmaterial gepackt ist, wie zum Beispiel Glas- oder Plastikperlen oder einem porösen Material, einschließlich einer immobilisierten DNA-Telomersequenz, die ausgewählt ist aus der Gruppe bestehend aus einer einzelsträngigen Telomersequenz, einem dazu komplementären Strang, einem doppelsträngigen Telomer, einem Teil oder einer Wiederholung oder einer Kombination der vorher genannten, und die in der Flüssigkeit befindliche anti-DNA-Autoantikörper binden kann.

33. Festphasenmittel nach Anspruch 32, wobei die Telomersequenz ausgewählt ist aus der Gruppe bestehend aus einem Vertebratentelomerstrang 5'-TTAGGG-3', dem dazu komplementären Strang, einem doppelsträngigen Vertebratentelomer, einem funktionellen Teil von einem der Stränge, einer Wiederholung von einem der Stränge und einer Kombination der vorher genannten.

34. Festphasenmittel nach Anspruch 33, wobei die Wiederholung des Telomerstranges ausgewählt ist aus der Gruppe bestehend aus und einem Duplex der beiden Stränge.

35. Festphasenmittel nach Anspruch 32, wobei die Telomersequenz am 3'- oder am 5'- Ende biotinyliert und an einem Streptavidin-beschichteten Träger immobilisiert ist.

36. Verwendung von Telomersequenzen oder Fragmenten von oder Antikörpern davon, wobei die Telomersequenz ausgewählt ist aus der Gruppe bestehend aus einer einzelsträngigen Telomersequenz eines Vertebraten, einem dazu komplementären Strang, einem doppelsträngigen Vertebraten-Telomer, und einem Teil oder einer Wiederholung oder einer Kombination der vorher genannten zur Herstellung eines Medikaments für die Behandlung von Autoimmunerkrankungen und/oder Krankheiten, die hervorgerufen werden durch anti-DNA-Autoantikörper oder diese beinhalten, wobei die Telomersequenz in dem Medikament an einen pharmazeutisch verträglichen Träger gebunden ist.

37. Verwendung nach Anspruch 36, wobei die Autoimmunkrankheit ausgewählt ist aus der Gruppe bestehend aus Lupus erythematodes, rheumatoider Arthritis und Sclerodermie.

38. Testkit zum Nachweis von anti-DNA-Autoantikörpern, insbesondere zur Diagnose von Autoimmunkrankheiten bei Vertebraten, **dadurch gekennzeichnet, dass** der Testkit eine nicht-immobilisierte DNA-Telomersequenz eines Vertebraten beinhaltet, die ausgewählt ist aus der Gruppe bestehend aus einer einzelsträngigen Telomersequenz eines Verbraten, einem dazu komplementären Strang, einem doppelsträngigen Telomer eines Vertebraten, einem Teil oder einer Wiederholung oder einer Kombination der vorher genannten, die in der Flüssigkeit des Vertebraten befindliche anti-DNA-Autoantikörper binden kann, und aus einem Marker, der die Bindung des anti-DNA-Autoantikörpers an die Sequenz anzeigen kann, und einem Mittel zum Nachweis der Bindung.

39. Verfahren zur Reinigung eines anti-Telomer-Antikörpers oder eines Fragments des anti-Telomer-Antikörpers aus einer Flüssigkeit durch Binden der anti-Telomer-Antikörpers in der Flüssigkeit an eine Telomersequenz, die ausgewählt ist aus der Gruppe bestehend aus einer einzelsträngigen Telomersequenz, einem dazu komplementären Strang, einem doppelsträngigen Telomer und einem Teil oder einer Wiederholung oder einer Kombination der vorher genannten, die an einer Affinitätssäule immobilisiert ist.

## Revendications

1. Procédé de détection d'auto-anticorps anti-ADN dans un échantillon, **caractérisé par** la mise en contact dudit échantillon avec une séquence télomère d'ADN choisie dans un ensemble constitué d'une séquence télomère simple brin, d'un brin complémentaire de celle-ci, d'un télomère double brin, et d'une partie ou d'une répétition ou d'une combinaison de tous ceux qui précèdent, pour fournir la liaison desdits auto-anticorps à ladite séquence télomère d'ADN.

2. Procédé selon la revendication 1, dans lequel la séquence télomère est choisie dans l'ensemble constitué du brin télomère de vertébré 5'-TTAGGG-3', du brin complémentaire de celui-ci, d'un télomère double brin de vertébré, d'une partie fonctionnelle de l'un quelconque desdits brins, d'une répétition ou de l'un quelconque desdits brins et d'une combinaison de tous ceux qui précèdent.

3. Procédé selon la revendication 2, dans lequel ladite répétition dudit brin télomère est choisie dans l'ensemble constitué de et d'un duplex de ceux-ci.

4. Procédé selon la revendication 1, dans lequel on utilise un anticorps dirigé contre ladite séquence télomère ou un fragment dudit anticorps, en particulier un fragment F(ab')₂ ou un fragment Fab, dans ladite détection.

5. Procédé selon la revendication 4, dans lequel la classe d'immunoglobulines (Ig) desdits auto-anticorps anti-ADN est déterminée à l'aide d'anticorps anti-Ig, en particulier d'anticorps anti-IgG, anti-IgM ou anti-IgA.

6. Procédé selon la revendication 5, dans lequel la sous-classe d'immunoglobulines desdits auto-anticorps anti-ADN est déterminée à l'aide d'anticorps anti-Ig d'une sous-classe spécifique, en particulier d'anticorps anti-IgG1 ou anti-IgG2.

7. Procédé selon la revendication 1, dans lequel ledit échantillon est un fluide corporel de vertébré.

8. Procédé selon la revendication 7, dans lequel le fluide corporel est du sérum humain ou du fluide cérébrospinal.

9. Procédé selon la revendication 7 ou 8 pour diagnostiquer des maladies auto-immunes chez des vertébrés, dans lequel l'échantillon de fluide corporel dudit vertébré est amené au contact de ladite séquence télomère d'ADN du vertébré, un taux élevé d'auto-anticorps anti-ADN présents dans ledit fluide corporel étant considéré comme étant indicatif d'une telle maladie chez ledit vertébré.

10. Procédé selon la revendication 9, dans lequel ladite maladie auto-immune est choisie dans l'ensemble constitué du lupus érythémateux, de l'arthrite rhumatoide et de la sclérodermie.

11. Procédé selon la revendication 10, dans lequel ladite maladie auto-immune est le lupus érythémateux systémique (LES).

12. Procédé selon la revendication 9, dans lequel ladite séquence télomère est choisie dans l'ensemble constitué du brin télomère 5'-TTAGGG-3' de vertébré, du brin complémentaire de celui-ci, d'un télomère double brin de vertébré, d'une partie fonctionnelle de l'un quelconque desdits brins, d'une répétition de l'un quelconque desdits brins et d'une combinaison de tous ceux qui précèdent.

13. Procédé selon la revendication 12, dans lequel ladite répétition dudit brin télomère est choisie dans l'ensemble constitué de et d'un duplex de ceux-ci.

14. Procédé selon la revendication 9, dans lequel on utilise un anticorps ou un fragment d'un anticorps dirigé contre ladite séquence télomère, en particulier un fragment F(ab')₂ ou un fragment Fab, dans ledit diagnostic.

15. Procédé selon la revendication 14, dans lequel la classe d'immunoglobulines (Ig) desdits anticorps anti-ADN est déterminée à l'aide d'anticorps anti-Ig, en particulier d'anticorps anti-IgG, anti-IgM ou anti-IgA.

16. Procédé selon la revendication 15, dans lequel on détecte un LES chez un patient humain en amenant un échantillon dudit fluide corporel du patient au contact d'une séquence télomère immobilisée d'un vertébré pour lier des auto-anticorps anti-ADN présents dans ledit fluide à ladite séquence télomère immobilisée et en détectant un taux élevé d'auto-anticorps liés de la classe des IgG parmi lesdits anticorps anti-ADN liés.

17. Procédé selon la revendication 15, dans lequel on détecte une arthrite rhumatoide chez un patient humain en amenant un échantillon dudit fluide corporel du patient au contact d'une séquence télomère immobilisée de vertébré pour lier des auto-anticorps anti-ADN présents dans ledit sérum à ladite séquence télomère immobilisée et en détectant un taux élevé d'auto-anticorps liés de la classe des IgM parmi lesdits auto-anticorps liés anti-ADN.

18. Coffret (« kit ») d'essai pour la détection d'auto-anticorps anti-ADN, en particulier pour le diagnostic de maladies auto-immunes chez des vertébrés, **caractérisé en ce que** ledit coffret d'essai comprend une séquence télomère immobilisée d'ADN de vertébré, choisie dans l'ensemble constitué d'une séquence télomère simple brin de vertébré, d'un brin complémentaire de celle-ci, d'un télomère double brin de vertébré, d'une partie ou d'une répétition ou d'une combinaison de tous ceux qui précèdent, capable de lier des auto-anticorps anti-ADN présents dans un fluide corporel dudit vertébré, ainsi qu'un marqueur capable d'indiquer la liaison ou l'inhibition de la liaison dudit auto-anticorps anti-ADN à ladite séquence.

19. Coffret d'essai selon la revendication 18, comprenant un anticorps ou un fragment d'un anticorps dirigé contre ladite séquence télomère, en particulier un fragment F(ab')₂ ou un fragment Fab, à utiliser dans ladite détection.

20. Coffret d'essai selon la revendication 19, comprenant des anticorps spécifiques anti-Ig, en particulier des anticorps anti-IgG, anti-IgM ou anti-IgA, à utiliser pour déterminer la classe d'immunoglobulines (Ig) desdits auto-anticorps anti-ADN.

21. Coffret d'essai selon la revendication 20, comprenant des anticorps spécifiques anti-Ig d'une sous-classe, en particulier des anticorps anti-IgG1 ou anti-IgG2, pour la détection de sous-classes d'immunoglobulines desdits auto-anticorps anti-ADN.

22. Coffret d'essai selon la revendication 18, dans lequel ladite maladie auto-immune est choisie dans l'ensemble constituée du lupus érythémateux, de l'arthrite rhumatoïde et de la sclérodermie.

23. Coffret d'essai selon la revendication 22, dans lequel ladite maladie auto-immune est le lupus érythémateux systémique (LES).

24. Coffret d'essai selon la revendication 18, dans lequel ladite séquence télomère est choisie dans l'ensemble constituée du brin télomère 5'-TTAGGG-3' de vertébré, du brin complémentaire de celui-ci, d'une partie fonctionnelle de l'un quelconque desdits brins, d'une répétition de l'un quelconque desdits brins et d'une combinaison de tous ceux qui précèdent.

25. Coffret d'essai selon la revendication 24, dans lequel ladite répétition dudit brin télomère est choisie dans l'ensemble constitué de et d'un duplex de celles-ci.

26. Coffret d'essai selon la revendication 18, dans lequel ladite séquence télomère est biotinylée à l'extrémité 3' ou 5' et est immobilisée sur un support revêtu de streptavidine.

27. Coffret d'essai selon la revendication 19, dans lequel ledit marqueur est fourni sur un anticorps anti-Ig spécifique du télomère, sur un fragment de celui-ci ou sur une seconde séquence télomère.

28. Coffret d'essai selon la revendication 19, dans lequel ledit marqueur est choisi dans l'ensemble constitué d'un marqueur radioactif, d'un marqueur enzymatique, d'un marqueur fluorochrome ou de fluorescence, d'un colorant, de biotine, d'un marqueur luminescent et d'une particule colorée comme une particule colorée de latex.

29. Coffret d'essai selon la revendication 19, comprenant une quantité d'un ou plusieurs anticorps anti-Ig marqués choisis dans l'ensemble constitué d'anticorps anti-IgG, anti-IgM, anti-IgA, anti-IgD et anti-IgE ou des sous-classes de ceux-ci.

30. Coffret d'essai selon la revendication 29, dans lequel ledit anticorps anti-Ig est choisi dans l'ensemble constitué d'anticorps anti-IgG, anti-IgM, anti-IgA, anti-IgG1 et anti-IgG2.

31. Coffret d'essai selon la revendication 18 ou 26, dans lequel ladite séquence télomère est immobilisée sur les parois de puits de microtitrage, sur du Sépharose, sur des perles de verre ou de matière plastique ou sur une feuille poreuse.

32. Moyen en phase solide pour la séparation d'auto-anticorps anti-ADN à partir d'un échantillon de fluide, ledit moyen se présentant sous la forme d'une colonne d'affinité garnie d'une matière solide, comme des perles de verre ou de matière plastique ou une matière poreuse, comprenant une séquence télomère immobilisée d'ADN choisie dans l'ensemble constitué d'une séquence télomère simple brin, d'un brin complémentaire de celle-ci, d'un télomère double brin, d'une partie ou d'une répétition ou d'une combinaison de tous ceux qui précèdent, capable de lier des auto-anticorps anti-ADN présents dans un fluide.

33. Moyen en phase solide selon la revendication 32, dans lequel ladite séquence télomère est choisie dans l'ensemble constitué du brin télomère 5'-TTAGGG-3' de vertébré, du brin complémentaire de celui-ci, d'un télomère double brin de vertébré, d'une partie fonctionnelle de l'un quelconque desdits brins, d'une répétition de l'un quelconque desdits brins et d'une combinaison de tous ceux qui précèdent.

34. Moyen en phase solide selon la revendication 33, dans lequel ladite répétition dudit brin télomère est choisie dans l'ensemble constitué de et d'un duplex de celles-ci.

35. Moyen en phase solide selon la revendication 32, dans lequel ladite séquence télomère est biotinylée à l'extrémité 3' ou 5' et est immobilisée sur un support revêtu de streptavidine.

36. Utilisation de séquences télomères ou de fragments d'anticorps dirigés contre celles-ci, ladite séquence télomère étant choisie dans l'ensemble constitué d'une séquence télomère simple brin de vertébré, d'un brin complémentaire de celle-ci, d'un télomère double brin de vertébré, et d'une partie ou d'une répétition ou d'une combinaison de tous ceux qui précèdent, pour la fabrication d'un médicament pour le traitement de dysfonctionnements auto-immuns et/ou de maladies causés par ou comprenant la présence d'auto-anticorps anti-ADN, ladite séquence télomère étant incluse dans le médicament avec un véhicule pharmaceutique acceptable.

37. Utilisation selon la revendication 36, dans laquelle ladite maladie auto-immune est choisie dans l'ensemble constitué du lupus érythémateux, de l'arthrite rhumatoïde et de la sclérodermie.

38. Coffret d'essai pour la détection d'auto-anticorps anti-ADN, en particulier pour le diagnostic de maladies auto-immunes chez des vertébrés, **caractérisé en ce que** ledit coffret d'essai comprend une séquence télomère non immobilisée d'ADN de vertébré, choisie dans l'ensemble constitué d'une séquence télomère simple brin de vertébré, d'un brin complémentaire de celle-ci, d'un télomère double brin de vertébré, d'une partie ou d'une répétition ou d'une combinaison de l'un quelconque des précédents, capable de lier des auto-anticorps anti-ADN présents dans un fluide corporel dudit vertébré, ainsi qu'un marqueur capable d'indiquer la liaison dudit auto-anticorps anti-ADN à ladite séquence, et un moyen pour détecter la liaison.

39. Procédé de purification d'un anticorps anti-télomère à partir d'un fluide en liant ledit anticorps anti-télomère dans ledit fluide à une séquence télomère choisie dans l'ensemble constitué d'une séquence télomère simple brin, d'un brin complémentaire de celle-ci, d'un télomère double brin et d'une partie ou d'une répétition ou d'une combinaison de l'un quelconque des précédents, immobilisée dans une colonne d'affinité, ou d'un fragment dudit anticorps anti-télomère.
